# EUROPEAN PATENT SPECIFICATION

(11) **EP 4 504 342 B1**
(45) Date of publication and mention of the grant of the patent: **29.04.2026**
(21) Application number: 23717851.2
(22) Date of filing: 31.03.2023
(51) Int. Cl.: A61P 31/14, C07K 14/435, A61K 38/05, A61K 38/16

(54) **BIFUNCTIONAL PEPTIDE WITH MUCOADHESIVE AND VIRUS BINDING PROPERTIES**
BIFUNKTIONELLES PEPTID MIT MUCOADHÄSIVEN UND VIRUSBINDENDEN EIGENSCHAFTEN
PEPTIDE BIFONCTIONNEL AVEC PROPRIÉTÉS MUCOADHÉSIVES ET VIRALES

(30) Priority: 01.04.2022 EP 22166380
(43) Date of publication of application: 12.02.2025
(73) Proprietor: Freie Universität Berlin, 14195 Berlin (DE); Forschungsverbund Berlin E.V., 12489 Berlin (DE)
(72) Inventor: LAUSTER, Daniel, 10179 Berlin (DE); DIEHN, Robyn, 21442 Toppenstedt (DE); POVOLOTSKY, Tatyana, 12524 Berlin (DE); BARDUA, Markus, 10439 Berlin (DE); HACKENBERGER, Christian, 10777 Berlin (DE); FLOREZ, Sebastian, 10407 Berlin (DE); MALY, Jan, 25166 Hrusice (CZ); HERRMANN, Andreas, 14467 Potsdam (DE)
(74) Representative: Maikowski & Ninnemann Patentanwälte Partnerschaft mbB
(86) International application number: PCT/EP2023/058432
(87) International publication number: WO 2023/187142

(56) References cited:
- WO-A1-2021/190980
- WO-A1-2021/257781
- DANIEL LAUSTER ET AL: "Multivalent Peptide-Nanoparticle Conjugates for Influenza-Virus Inhibition", ANGEWANDTE CHEMIE, VERLAG CHEMIE, HOBOKEN, USA, vol. 56, no. 21, 26 April 2017 (2017-04-26), pages 5931 - 5936, XP072093868, ISSN: 1433-7851, DOI: 10.1002/ANIE.201702005
- HOFFMANN WERNER: "Trefoil Factor Family (TFF) Peptides and their Different Roles in the Mucosal Innate Immune Defense and More: An Update", vol. 28, no. 36, 1 November 2021 (2021-11-01), NL, pages 7387 - 7399, XP055968311, ISSN: 0929-8673, Retrieved from the Internet <URL:https://www.eurekaselect.com/191403/article> DOI: 10.2174/0929867328666210215114140
- HAN D P ET AL: "Identification of critical determinants on ACE2 for SARS-CoV entry and development of a potent entry inhibitor", VIROLOGY, ELSEVIER, AMSTERDAM, NL, vol. 350, no. 1, 20 June 2006 (2006-06-20), pages 15 - 25, XP024896563, ISSN: 0042-6822, [retrieved on 20060620], DOI: 10.1016/J.VIROL.2006.01.029
- MUSKETT FREDERICK W. ET AL: "Solution Structure of the Disulfide-Linked Dimer of Human Intestinal Trefoil Factor (TFF3):? The Intermolecular Orientation and Interactions Are Markedly Different from Those of Other Dimeric Trefoil Proteins", BIOCHEMISTRY, vol. 42, no. 51, 1 December 2003 (2003-12-01), pages 15139 - 15147, XP055968359, ISSN: 0006-2960, DOI: 10.1021/bi030182k

## Description

The present invention relates to a bifunctional peptide according to the preamble of claim 1, to the medical use of such a peptide according to the preamble of claim 8, to a medicament comprising such a peptide according to the preamble of claim 11, and to a method for manufacturing such a peptide according to the preamble of claim 13.

The ongoing SARS-CoV-2 pandemic exemplified that emerging and recurring viral pathogens are a constant threat to the human population, requiring a fast and specific response to contain extensive viral spread. Despite the quick implementation of hygiene concepts to slow down viral transmission, vaccines and antiviral therapy are the main measures against infectious diseases. However, the development and testing of vaccines often requires several years. Antiviral therapy provides a quick solution to treat already infected individuals. Small organic compounds dominate the group of antivirals as opposed to larger molecules such as proteins or peptides. However, the development of new small molecule drugs is challenging due to their often complex chemistry, and thus difficult to prepare screenings with a large variety of different compound analogues. In addition, the development of resistance is a growing problem as evidenced for antiviral drugs against influenza such as oseltamivir or amantadine.

Prophylactic treatment as an additional measure against the SARS-CoV-2 pandemic is currently not used, a missed opportunity that could bring several advantages.

Werner Hoffmann (Hoffmann, Werner. "Trefoil factor family (TFF) peptides and their different roles in the mucosal innate immune defense and more: An update." Current Medicinal Chemistry 28.36 (2021): 7387-7399) describes a heterodimer from trefoil factor (TFF3) and IgG-Fc-binding protein (FCGBP).

Lauster et al. (Lauster, Daniel, et al. "Multivalent peptide-nanoparticle conjugates for influenza-virus inhibition." Angewandte Chemie International Edition 56.21 (2017): 5931-5936.) describes multivalent peptide-polymer nanoparticles binding with nanomolar affinity to influenza virus via its spike protein hemagglutinin. The authors were able to demonstrate in vitro that by increasing the size of the polymer scaffold and adjusting the peptide density, viral infection was drastically reduced.

It is an object of the present invention to provide novel substances being useful in prophylactic treatment of viral diseases, ideally avoiding a viral infection.

This object is achieved with a peptide having the claim elements of claim 1. Such a peptide is a bifunctional peptide that comprises a virus binding moiety and a mucin binding moiety that is covalently bound to the virus binding moiety. The binding between the virus binding moiety and the mucin binding moiety can be a direct covalent binding or a binding via a linker.

The virus binding moiety comprises or is a peptide being at least 95 %, in particular at least 96 %, in particular at least 97 %, in particular at least 98 %, in particular at least 99 %, in particular 100 %, identical to SEQ ID NO. 48.

SEQ ID NO. 48 describes a peptide particularly appropriate for binding the influenza A virus (in particular strains H1, H3, H7). This peptide targets the hemagglutinin (HA protein) of the influenza A virus and is described by Memczak et al. and Miriam Hoffmann et al.

SEQ ID NO. 2 to 13 and 23 to 43 as disclosed herein but not forming part of the claimed invention relate to virus binding moieties that are particularly appropriate for binding the severe acute respiratory syndrome coronavirus 2 (SARS-CoV-2). While SEQ ID NO. 2, 3, and 23 to 41 relate to linear virus binding peptides, SEQ ID NO. 4 to 13, 42, and 43 relate to partially cyclized peptides, so-called stapled peptides. They comprise at least one bridge between two not directly subsequent (but yet nearby) amino acids. This bridge acts like a staple and serves for a stabilization of the respective peptide. The bridge can be established by the two side chains of the respective amino acid residues or by a chemical moiety being covalently bound to the respective amino acid residues in addition to their regular side chains. In case of SEQ ID NO. 42 and 43, the bridge is a disulfide bridge formed between two cysteine residues.

SEQ ID NO. 49 and 50 as disclosed herein but not forming part of the claimed invention describe peptides particularly appropriate for binding the influenza A virus (in particular strain H5N1 clade 2.3.4, bird strain). These peptides target the hemagglutinin (HA protein) of the influenza A virus and are derived from sequences of antibody 65C6 described by Zuo et al.

SEQ ID NO. 51 and 52 as disclosed herein but not forming part of the claimed invention describe peptides particularly appropriate for binding the influenza A virus (in particular strain H5N1 clade 2.3.2.1, bird strain). These peptides target specific sequences of the hemagglutinin (HA protein) found in general in HA protein of variants of this strain, for example in the sequences of the HA protein described by Bui et al.

SEQ ID NO. 53 and 54 as disclosed herein but not forming part of the claimed invention describe peptides particularly appropriate for binding the influenza B virus. These peptides target the neuraminidase of the influenza B virus and are described by Madsen et al.

SEQ ID NO. 55 and 56 as disclosed herein but not forming part of the claimed invention describe peptides particularly appropriate for binding rhinoviruses (RVs) and other enteroviruses (EV). These peptides are derived from anti-RV antibodies that are described by Hrebik et al., Khan et al., and Smith et al.

SEQ ID NO. 57 as disclosed herein but not forming part of the claimed invention describes a peptide particularly appropriate for binding the human parainfluenza virus (HPIV) (in particular strains HIPV1 and HIPV3). This peptide targets the fusion protein (F protein) of HPIV and is derived from anti-HPIV antibodies described by Cabán et al.

SEQ ID NO. 58, 59, and 60 as disclosed herein but not forming part of the claimed invention describe peptides particularly appropriate for binding the metapneuvirus (MPV) and the respiratory syncytial virus (RSV) (in particular subtype A and/or subtype B). These peptides target the fusion protein (F protein) of MPV and of RSV. They are derived from anti-HPIV antibodies and are described by Cabán et al.

SEQ ID NO. 61, 62, and 63 as disclosed herein but not forming part of the claimed invention describe peptides particularly appropriate for binding RSV (in particular subtype A and/or subtype B). These peptides also target the fusion protein (F protein) of RSV and are described by Issmail et al, Zhu et al., and McLellan et al.

The mucin binding moiety comprises or is a peptide being at least 95 %, in particular at least 96 %, in particular at least 97 %, in particular at least 98 %, in particular at least 99 %, in particular 100 %, identical to SEQ ID NO. 1 (modified trefoil factor 3, TFF3 (C57K)), SEQ ID NO. 14 (native TFF3), SEQ ID NO. 20 (native TFF1), SEQ ID NO. 21 (native TFF2), or SEQ ID NO. 22 (jacalin). These lectins are particularly appropriate to target a patient's intestinal mucosa. TFF3 is a naturally produced protein in the airways (Thim et al. 2002).

The presently claimed peptide is able to strengthen the natural barrier function of respiratory mucus. Respiratory mucus is the first physical barrier encountered by respiratory pathogens approaching their target cells of the respiratory epithelium. With its high viscosity and microbial-binding properties, mucus is an important part of our body's antimicrobial defense system and efficiently clears adsorbed pathogens through ciliary movement. The presently claimed peptide can be used to strengthen the mucus by increasing its antimicrobial properties through the incorporation of binding sites for viral pathogens (mucus augmentation). Retained pathogens can then be cleared before reaching the epithelial cell layer, thus preventing infection. The present approach is thus to use modular biomimetic peptides to increase and support the antiviral efficacy of mucus.

Mucus augmentation is a new concept for protecting people against respiratory infections. The present approach can be combined with other (non)-pharmacological measures to reduce the risk of infection in uninfected individuals and the risk of transmission and severe disease in already infected ones.

The presently claimed peptide constitutes the core of an adjustable peptide-based system with a molecule that can adhere to airway mucins and specifically absorb viruses. Both parts of the peptide can be adapted to respond to a quickly changing situation. This biomimetic approach reinforces the natural antiviral properties of mucus, and as the peptide components are derived from human proteins, side-effects are considered to be low. The bifunctional peptide can also be used therapeutically in infected patients, as it will also trap viruses released from infected cells, thereby reducing the viral titer and shedding into the environment. Finally, the present approach can be combined with established antiviral measures, together helping to reduce the risk of infection and minimizing the risk of disease.

Due to the modular composition of the claimed peptide, both functional moieties of the peptide can be adapted for the desired application.

An appropriate linker for establishing a covalent binding between the mucin binding moiety and the virus binding moiety is a polyethylene glycol (PEG) linker or an amino acid linker comprising 1 to 20 amino acids, in particular 2 to 19 amino acids, in particular 3 to 18 amino acids, in particular 4 to 17 amino acids, in particular 5 to 16 amino acids, in particular 6 to 15 amino acids, in particular 7 to 14 amino acids, in particular 8 to 13 amino acids, in particular 9 to 12 amino acids, in particular 10 to 11 amino acids. A dipeptide, such as a GT dipeptide, is a particularly appropriate linker. A linker comprising or consisting of an amino acid sequence being at least 95 %%, in particular at least 96 %, in particular at least 97 %, in particular at least 98 %, in particular at least 99 %, in particular 100 % identical to SEQ ID NO. 44, SEQ ID NO. 45, SEQ ID NO. 46, or SEQ ID NO. 47 it is a particularly appropriate linker, too. SEQ ID NO. 44 is an example for a flexible linker. SEQ ID NO. 45 is an example for a rigid linker. SEQ ID NO. 46 represents a linking peptide resulting from a chemo-enzymatic coupling reaction via Sortase A. SEQ ID NO. 47 represents a linking peptide resulting from a chemo-enzymatic coupling reaction via transglutaminase. The virus binding moiety or the mucin binding moiety peptide is coupled to the glutamyl residue (Q7) via a γ-glutamyl-ε-lysyl isopeptide bond.

In an embodiment, the mucin binding moiety comprises a glycotope binding domain and binds to an N-acetylglucosamine-alpha-1,4-galactose (GlcNAc-α-1,4-Gal) disaccharide with an affinity of at least 150 µM, in particular of at least 100 µM, in particular of at least 75 µM, in particular of at least 50 µM, in particular of at least 25 µM, in particular with an affinity lying in a range of from 25 µM to 150 µM, in particular from 50 µM to 125 µM, in particular from 75 µM to 100 µM. The GlcNAc-α-1,4-Gal disaccharide is a glycotope that is specifically present in mucins such as mucin 2 (Muc2), mucin 5AC (Muc5AC), and mucin 6 (Muc6). To give an example, natural trefoil factor 3 (TFF3) has a binding affinity towards the GlcNAc-α-1,4-Gal disaccharide of 56 µM (Järvå et al 2020).

In an embodiment, the virus binding moiety is covalently bound to the mucin binding moiety at amino acid 57 of the mucin binding moiety. In an embodiment, amino acid 57 is a cysteine residue. In an embodiment, amino acid 57 is a lysine residue. In an embodiment, the virus binding moiety is covalently bound to the mucin binding moiety at the C terminus of the mucin binding moiety. In particular in the latter embodiment, it is of no special importance for the binding ability to the virus binding moiety which amino acid is present at position 57 (or at any other position) of the mucin binding moiety.

In an embodiment, the peptide comprises at least two virus binding moieties. In this context, the mucin binding moiety is directly or via a linker covalently bound to at least one of the at least two virus binding moieties. The mucin binding moiety may also be bound to more than one virus binding moieties. For this purpose, a bifurcated linker is, in an embodiment, particularly helpful. Such a bifurcated linker may be used to couple a single mucin binding moiety to a plurality of virus binding moieties that are, after coupling, arranged more or less equally distant from the mucin binding moiety.

Herewith disclosed but not forming part of the claimed invention is the possibility that the virus binding moiety is a moiety that is adapted to bind a viral spike protein.

In an embodiment, the virus binding moiety is a peptide. In an embodiment, the virus binding moiety is an antimicrobial peptide, such as a defensin. Retrocyclin is a particular appropriate defensin. Herewith disclosed but not forming part of the claimed invention is the possibility that the virus binding moiety is a non-peptidic antiviral residue, such as a dendritic or linear polysulfate or polycarbohydrate.

In an embodiment, the virus binding moiety is a peptide, wherein at least one of the residues of this peptide carries a substitution. In an embodiment, the C terminus of the peptide is substituted with an amino group. In an embodiment, the N terminus of the peptide is substituted with an acetyl group. In an embodiment, the C terminus of the peptide is substituted with an amino group and the N terminus of the peptide is substituted with an acetyl group.

Herewith disclosed but not forming part of the claimed invention is the possibility that the virus binding moiety is a peptide being at least 95 %, in particular at least 96 %, in particular at least 97 %, in particular at least 98 %, in particular at least 99 %, in particular 100 %, identical to SEQ ID NO. 2 and the mucin binding moiety (1) is a trefoil factor 3 peptide being at least 95 %%, in particular at least 96 %, in particular at least 97 %, in particular at least 98 %, in particular at least 99 %, in particular 100 %, identical to SEQ ID NO. 1. SEQ ID NO. 2 describes a peptide denoted p4 that was already shown in the past to bind to the severe acute respiratory syndrome coronavirus 1 (SARS-CoV-1) with high affinity (Han et al., 2006). Despite of structural differences between SARS-CoV-1 and SARS-CoV-2, it was surprisingly found that the p4 peptide is also very appropriate to bind to SARS-CoV-2.

Herewith disclosed but not forming part of the claimed invention is the possibility that the virus binding moiety is a peptide being at least 95 %, in particular at least 96 %, in particular at least 97 %, in particular at least 98 %, in particular at least 99 %, in particular 100 %, identical to SEQ ID NO. 3 and the mucin binding moiety (1) is a trefoil factor 3 peptide being at least 95 %%, in particular at least 96 %, in particular at least 97 %, in particular at least 98 %, in particular at least 99 %, in particular 100 %, identical to SEQ ID NO. 1. SEQ ID NO. 3 describes a peptide denoted p6 that was already shown in the past to bind to the severe acute respiratory syndrome coronavirus 1 (SARS-CoV-1) with high affinity (Han et al., 2006). Despite of structural differences between SARS-CoV-1 and SARS-CoV-2, it was surprisingly found that the p6 peptide is also very appropriate to bind to SARS-CoV-2. Thereby, the binding affinity of the p6 peptide with respect to SARS-CoV-2 is approximately twice as high as the affinity of the p4 peptide. Interestingly, p6 is also able to bind SARS-CoV-2 variants alpha and delta with an even higher affinity than the SARS-CoV-2 Wuhan wild type (cf. Table 1).

An even higher affinity towards SARS-CoV-2 than that of the linear p6 peptide was observed for stapled variants of the p6 peptide. Herewith disclosed but not forming part of the claimed invention is the possibility that, the virus binding moiety is a peptide being at least 95 %, in particular at least 96 %, in particular at least 97 %, in particular at least 98 %, in particular at least 99 %, in particular 100 %, identical to SEQ ID NO. 10. This peptide is a stapled variant of the p6 peptide. In the following, it is also referred to as p6-cyc or p6 (R8)₁₅(S5)₂₂.

The binding affinity of different virus binding moieties towards SARS-CoV-2 (expressed as the dissociation constant Kd) is summarized in the following Table 1. It was determined by microscale thermophoresis (MST). It can be well seen that the p4 and the p6 peptides (the latter both in its linear in its cyclized form) bind to several receptor binding domains of currently circulating SARS-CoV-2 variants with nanomolar binding constants.

**Table 1: Properties of different virus binding moieties.**

| **Peptide name** | **Sequence ^{*1}** | **SEQ ID NO.** | **SARS-CoV-2 target strain** | **Kd/µM** | **Helicity in PBS/% ^{*2}** | **Helicity in PBS:TFE, 1:1 ^{*3}** |
|---|---|---|---|---|---|---|
| p4 | EEQAKTFLDKFNHEA EDLFYQSS | 2 | 2019-nCov | 0.93 ± 0.21 | 0 | 100 |
| p6 | EEQAKTFLDKFNHEA EDLFYQSSGLGKGDF R | 3 | 2019-nCov | 0.45 ± 0.10 | 0 | 39 |
| p6-cyc | EEQAKTFLDKFNHEX EDLFYQXSGLGKGDF R | 10 | 2019-nCov | 0.27 ± 0.14 | 25 | 43 |
| p6 | see above | 3 | B.1.1.7 (alpha) | 0.32 ± 0.06 | 0 | 39 |
| p6 | see above | 3 | B.1.617.2 (delta) | 0.19 ± 0.09 | 0 | 39 |
| p6 | see above | 3 | B.1.1.529 (omicron) | 0.28 | 0 | 39 |

| | | | | | | |
|---|---|---|---|---|---|---|
| ^{*1} X in position 15 (X₁₅) of p6-cyc is an alkenylated (R)-Ala residue; X in position 22 (X₂₂) of p6-cyc is an alkenylated (S)-Ala residue; the peptide has a cyclization between X₁₅ and X₂₂ via a 7-undecenyl residue. ^{*2} PBS = phosphate-buffered saline ^{*3} TFE = trifluoroethanol | | | | | | |

Herewith disclosed but not forming part of the claimed invention is the possibility that the peptide is at least 95 %, in particular at least 96 %, in particular at least 97 %, in particular at least 98 %, in particular at least 99 %, in particular 100 %, identical to SEQ ID NO. 18 or SEQ ID NO. 19. These SEQ ID NO. relate to synthetic and recombinant TFF-3-p6 peptides that turned out to have very appropriate properties for solving the present object.

In an aspect, the present invention relates to the use of the peptide according to the preceding explanations as medicament.

In an aspect, the present invention relates to the medical use of the peptide according to the preceding explanations in preventing or treating a viral infection.

In an embodiment, the viral infection to be treated or prevented is an infection with influenza A virus.

Herewith disclosed but not forming part of the claimed invention is a medical method for treating or preventing a viral infection in a patient in need of such treatment. The term "patient" as used herein relates to a human or animal patient. Mammals are particularly appropriate animal patients. The method comprises the step of administering the peptide according to the preceding explanations to the patient (e.g., in form of a medicament comprising this peptide). This administration is done, in an embodiment, by providing the peptide to the nose or to the mouth of the patient. This can be particularly easy achieved by providing the peptide in a dosage form of a spray. Then, the peptide can be administered, e.g., as an aerosol like a nasal spray or an inhalation spray. Upon administration, it will then contact the patient's mucosa and will allow a binding of the peptide to the mucosa via the mucin binding moiety. Afterwards, the peptide will stay in its bound form at the patient's mucosa and will be able to "filter" viruses out of the air inhaled by the patient due to its virus binding moiety.

Herewith disclosed but not forming part of the claimed invention is a medical method for mucus augmentation in a patient in need thereof. This method comprises the step of administering the peptide according to the preceding explanations to the patient (e.g., in form of a medicament comprising this peptide).

In an aspect, the present invention relates to a medicament that comprises a peptide according to the preceding explanations as pharmaceutically active ingredient. This medicament can comprise other pharmaceutically active ingredients. In an embodiment, the peptide is the only pharmaceutically active ingredient.

In an embodiment, the medicament is formulated such that it can be administered as an aerosol, such as a nasal spray or as an inhalation spray. Then, the medicament (and therewith the peptide as pharmaceutically active ingredient) directly contacts the mucosa in the nasopharyngeal zone upon its administration. This, in turn, leads to a mucus augmentation as explained above. As a result, the patient's mucus is better prepared to bind and subsequently eliminate viruses with which the patient is confronted (either by inhaling them from the environment or by exhaling them from virus-infected cells of the patient's body).

In an aspect, the present invention relates to a method for manufacturing a peptide according to the preceding explanations. This method comprises the steps explained in the following. First, a virus binding moiety precursor is reacted with a first mucin binding moiety precursor. In this context, the first mucin binding moiety precursor comprises a first part of the final mucin binding moiety. This reaction leads to the formation of a peptide precursor.

Afterwards, the peptide precursor is reacted with a second mucin binding moiety precursor. The second mucin binding moiety precursor comprises a second part of the mucin binding moiety. As a result of this second reaction step, the final peptide is formed. Of course, other intermediate reaction steps may also be accomplished. To give an example, it is possible to split the mucin binding moiety into more than two mucin binding moiety precursors. Then, the individual mucin binding moiety precursors are reacted one after the other with the already formed peptide precursor to finally result in the peptide.

In an embodiment, the virus binding moiety precursor comprises a 4-pentynoic moiety that establishes a covalent bond to the mucin binding moiety of the final peptide. This 4-pentynoic moiety may still be present (either fully or in part) in the final peptide. It may act as linker between the virus binding moiety and the mucin binding moiety in an embodiment.

In an embodiment, the peptide comprises a triazole residue acting as linker between the virus binding moiety and the mucin binding moiety. In an embodiment, the peptide comprises a polyethylene glycol residue acting as linker between the virus binding moiety and the mucin binding moiety. Such polyethylene glycol residue is combined with a triazole residue to form a linker in an embodiment.

In an embodiment, the first mucin binding moiety precursor comprises less than half of all amino acids of the mucin binding moiety. If only two mucin binding moiety precursors are used, then the second mucin binding moiety precursor comprises more than the half of all amino acids of the mucin binding moiety. To give a specific example, the first mucin binding moiety precursor comprises amino acids 36 to 59 of the mucin binding moiety, which is a trefoil factor 3 peptide in this embodiment. Furthermore, the second mucin binding moiety precursor comprises amino acids 1 to 35 of the mucin bending moiety. After having reacted the first mucin binding moiety precursor (corresponding to the C-terminal part of the mucin binding moiety) with the virus binding moiety, a reaction with the second mucin binding moiety precursor (corresponding to the N-terminal part of the mucin binding moiety) with the already formed construct of the first mucin binding moiety precursor and the virus binding moiety (i.e., the peptide precursor) is carried out. As a result, the final peptide is formed.

All embodiments of the peptide can be combined in any desired way and can be transferred either individually or in any arbitrary manner to the uses, to the medicament, and to the different methods. Likewise, all embodiments of the different uses can be combined in any desired way and can be transferred either individually or in any arbitrary manner to the peptide, to the respective other uses, to the medicament, and to the different methods. Likewise, all embodiments of the medicament can be combined in any desired way and can be transferred either individually or in any arbitrary manner to the peptide, to the different uses, and to the different methods. Furthermore, all embodiments of the different methods can be combined in any desired way and can be transferred either individually or in any arbitrary manner to the peptide, to the different uses, and to the respective other methods.

Further details of aspects of the present invention will be explained in the following making reference to exemplary embodiments and accompanying Figures. In the Figures:
- Figure 1A: schematically shows the structure of a first bifunctional peptide;
- Figure 1B: schematically shows the structure of a second bifunctional peptide;
- Figure 2A: shows the primary structure of an embodiment of a first mucin binding moiety precursor corresponding to SEQ ID NO. 15;
- Figure 2B: shows the primary structure of an embodiment of a second mucin binding moiety precursor corresponding to SEQ ID NO. 16;
- Figure 2C: shows the primary structure of a virus binding moiety precursor corresponding to SEQ ID NO. 17 (not forming part of the claimed invention);
- Figure 3: shows the primary structure of a peptide corresponding to SEQ ID NO. 18 (not forming part of the claimed invention) comprising a mucin binding moiety made from the first and second mucin binding moiety precursors of Figures 2A and 2B and a virus binding moiety made from the virus binding moiety precursor of Figure 2C;
- Figure 4: shows the primary structure of a virus binding moiety corresponding to SEQ ID NO. 4 (not forming part of the claimed invention);
- Figure 5: shows the primary structure of a virus binding moiety corresponding to SEQ ID NO. 5 (not forming part of the claimed invention);
- Figure 6: shows the primary structure of a virus binding moiety corresponding to SEQ ID NO. 6 (not forming part of the claimed invention);
- Figure 7: shows the primary structure of a virus binding moiety corresponding to SEQ ID NO. 7 (not forming part of the claimed invention);
- Figure 8: shows the primary structure of a virus binding moiety corresponding to SEQ ID NO. 8 (not forming part of the claimed invention);
- Figure 9: shows the primary structure of a virus binding moiety corresponding to SEQ ID NO. 9 (not forming part of the claimed invention);
- Figure 10: shows the primary structure of a virus binding moiety corresponding to SEQ ID NO. 10 (not forming part of the claimed invention);
- Figure 11: shows the primary structure of a virus binding moiety corresponding to SEQ ID NO. 11 (not forming part of the claimed invention);
- Figure 12: shows the primary structure of a virus binding moiety corresponding to SEQ ID NO. 12 (not forming part of the claimed invention);
- Figure 13: shows the primary structure of a virus binding moiety corresponding to SEQ ID NO. 13 (not forming part of the claimed invention);
- Figure 14: shows a plot illustrating the binding affinity between the p6 peptide and different SARS-CoV-2 variants;
- Figure 15: shows a plot illustrating the binding affinity between the bifunctional synthetic TFF3-p6 peptide corresponding to SEQ ID NO. 18 and different SARS-CoV-2 variants;
- Figure 16: shows a plot illustrating the binding affinity between the p4 peptide variant corresponding to SEQ ID NO. 23 and the receptor binding domain of SARS-CoV-2 (2019-nCoV);
- Figure 17: shows a plot illustrating the binding affinity between the p4 peptide variant corresponding to SEQ ID NO. 24 and the receptor binding domain of SARS-CoV-2 (2019-nCoV);
- Figure 18: shows a plot illustrating the binding affinity between the p4 peptide variant corresponding to SEQ ID NO. 25 and the receptor binding domain of SARS-CoV-2 (2019-nCoV);
- Figure 19: shows a plot illustrating the binding affinity between the p4 peptide variant corresponding to SEQ ID NO. 26 and the receptor binding domain of SARS-CoV-2 (2019-nCoV);
- Figure 20: shows a plot illustrating the binding affinity between the p4 peptide variant corresponding to SEQ ID NO. 28 and the receptor binding domain of SARS-CoV-2 (2019-nCoV);
- Figure 21: shows a plot illustrating the binding affinity between the p4 peptide variant corresponding to SEQ ID NO. 29 and the receptor binding domain of SARS-CoV-2 (2019-nCoV);
- Figure 22: shows a plot illustrating the binding affinity between the p4 peptide variant corresponding to SEQ ID NO. 30 and the receptor binding domain of SARS-CoV-2 (2019-nCoV);
- Figure 23: shows a plot illustrating the binding affinity between the p4 peptide variant corresponding to SEQ ID NO. 31 and the receptor binding domain of SARS-CoV-2 (2019-nCoV);
- Figure 24: shows a plot illustrating the binding affinity between the p4 peptide variant corresponding to SEQ ID NO. 32 and the receptor binding domain of SARS-CoV-2 (2019-nCoV);
- Figure 25: shows a plot illustrating the binding affinity between the p4 peptide variant corresponding to SEQ ID NO. 33 and the receptor binding domain of SARS-CoV-2 (2019-nCoV);
- Figure 26: shows a plot illustrating the binding affinity between the p4 peptide variant corresponding to SEQ ID NO. 34 and the receptor binding domain of SARS-CoV-2 (2019-nCoV);
- Figure 27: shows a plot illustrating the binding affinity between the p4 peptide variant corresponding to SEQ ID NO. 35 and the receptor binding domain of SARS-CoV-2 (2019-nCoV);
- Figure 28: shows a plot illustrating the binding affinity between the p4 peptide variant corresponding to SEQ ID NO. 36 and the receptor binding domain of SARS-CoV-2 (2019-nCoV);
- Figure 29: shows a plot illustrating the binding affinity between the p4 peptide variant corresponding to SEQ ID NO. 37 and the receptor binding domain of SARS-CoV-2 (2019-nCoV);
- Figure 30: shows a plot illustrating the binding affinity between the p4 peptide variant corresponding to SEQ ID NO. 38 and the receptor binding domain of SARS-CoV-2 (2019-nCoV);
- Figure 31: shows a plot illustrating the binding affinity between the p4 peptide variant corresponding to SEQ ID NO. 39 and the receptor binding domain of SARS-CoV-2 (2019-nCoV);
- Figure 32: shows a plot illustrating the binding affinity between the p4 peptide variant corresponding to SEQ ID NO. 40 and the receptor binding domain of SARS-CoV-2 (2019-nCoV);
- Figure 33: shows a plot illustrating the binding affinity between the p6 peptide variant corresponding to SEQ ID NO. 41 and the receptor binding domain of SARS-CoV-2 (2019-nCoV);
- Figure 34: shows a plot illustrating the binding affinity between the p6 peptide variant corresponding to SEQ ID NO. 42 and the receptor binding domain of SARS-CoV-2 (2019-nCoV);
- Figure 35: shows a plot illustrating the binding affinity between the p6 peptide variant corresponding to SEQ ID NO. 43 and the receptor binding domain of SARS-CoV-2 (2019-nCoV); and
- Figure 36: shows a plot illustrating the binding affinity between the bifunctional recombinant TFF3-p6 peptide corresponding to SEQ ID NO. 19 and different SARS-CoV-2 variants.

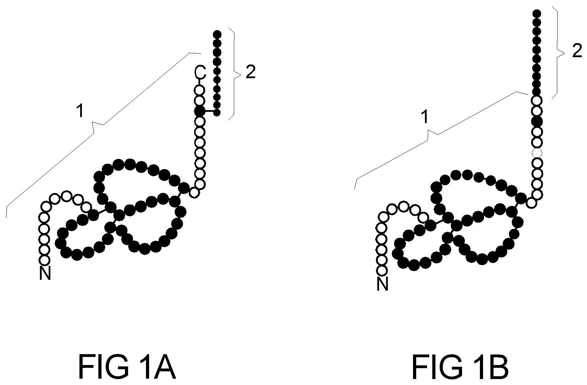

Figure 1A schematically illustrates the structure of a bifunctional peptide, namely TFF3-p6 (not forming part of the claimed invention). This is a peptide in which a trefoil factor 3 (TFF3) peptide 1 is the mucin binding moiety, wherein the p6 peptide 2 is the virus binding moiety. The TFF3 peptide 1 has a primary sequence corresponding to SEQ ID NO. 1. The p6 peptide 2 has a primary sequence corresponding to SEQ ID NO. 3. The p6 peptide 2 is covalently bound to lysine 57 of the TFF3 peptide.

Figure 1B shows an alternative bifunctional peptide (not forming part of the claimed invention). In this and in all following Figures, similar elements will be denoted with the same numeral reference. Here, the p6 peptide 2 is covalently bound to the C terminus of the TFF3 peptide 1.

The synthesis of TFF3-p6 will be explained in the following in more detail.

For the production of TFF3, the protocol of Braga Emidio et al. 2021 was followed. In contrast to the synthesis routes published by Braga Emidio et al., azido lysine was introduced into TFF3 by replacing cysteine at position 57.

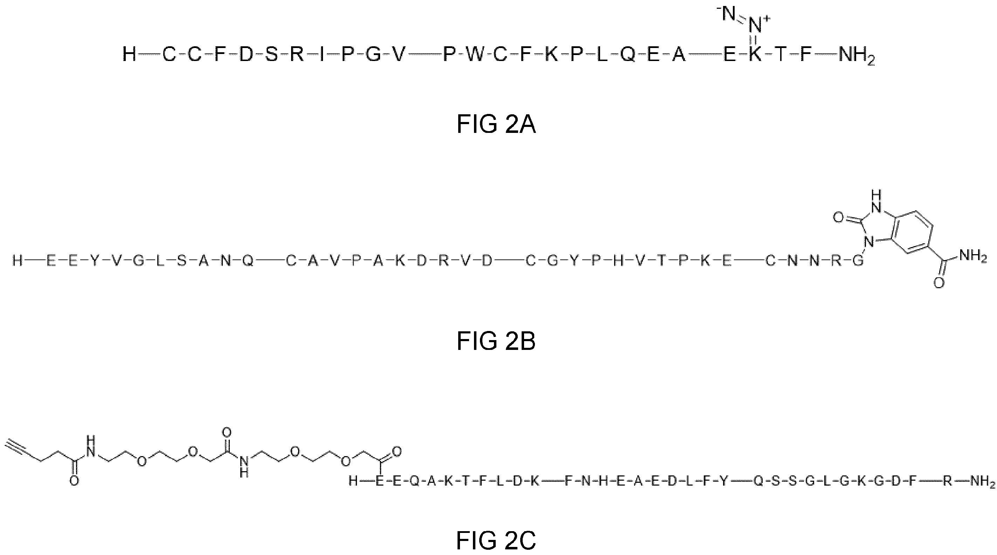

**TFF3(36-59)(C57AzK)** representing an embodiment of a first mucin binding moiety precursor was made totally by SPPS on Rink Amide Resin, using the corresponding building block for azido lysine (AzK) for position 57. TFF3₍₃₆₋₅₉₎C57AzK [M+H]⁺ = 2825.31 g/mol, [M+2H]³⁺/3 = 943.7 g/mol. The primary structure is illustrated in Figure 2A.

**TFF3(1-35)-Nbz** representing an embodiment of a second mucin binding moiety precursor was synthesized on Rink Amide Resin, which was initially functionalized with 3-fluorenylmethoxycarbonyl-4-diaminobenzoic acid (Fmoc-Dbz) by normal coupling methodologies (5 equivalents (eq) Fmoc-Dbz, 4.9 eq hexafluorophosphate azabenzotriazole tetramethyl uronium (HATU), 10 eq N,N-diisopropylethylamine (Dipea), in dimethylformamide (DMF) enough to make the solution 0.2 M regarding the 3-Fmoc-4-diaminobenzoic acid (Fmoc-Dbz). Then, normal solid phase peptide synthesis (SPPS) followed. The last residue was added as a Boc protected amino acid. Following the assembly of the peptide in the resin, the C-terminus was activated through acylation with p-nitrophenylchloroformate (5 eq, CH₂Cl₂, 45 min) followed by the addition of base (Dipea, 0.5 M in DMF 20 min) to promote intramolecular attack of the anilide to form the resin-bound benzimidazolinone. Cleavage from the resin was done using 94:2.5:2.5:1.0 of trifluoroacetic acid (TFA):1,2-Ethanedithiol (EDT):H₂O: Triisopropylsilane (TIPS), the crude was the dissolved in 3 mL of 30% Acetonitrile (MeCN)/H₂O with 0.1% TFA, filtered, and injected on a preparative high performance liquid chromatography (HPLC) system for purification. TFF3(1-35)-Nbz. 3980.4: M⁺, 996.1: (M+4H)⁴⁺/4. The primary structure is illustrated in Figure 2B.

**Pentynoic-(PEG)₂-p6** representing a virus binding moiety (not forming part of the claimed invention) was made by SPPS until the two PEG units, on a Rink Amide Resin. Then, 4-pentynoic acid was coupled by hand (5 eq 4-pentynoic acid, 4.9 eq HATU, 10 eq Dipea, in DMF enough to make the solution 0.2 M regarding the 4-pentynoic acid). Coupling was done twice for 1.5 hrs. Then, the peptide was cleaved with 95:2.5:2.5 of TFA:H₂O:TIPS, and purified by preparative HPLC. Penty-p6 [M+H]⁺ = 3975.90 g/mol, [M+2H]³⁺/3 = 994.9 g/mol. The primary structure is illustrated in Figure 2C.

In a small vial, CuSO₄ (10 eq) was added from a 1 mM solution, and mixed with THPTA (50 eq). The organometallic complex was allowed to form while mixing the other reagents.

To form a peptide precursor via click chemistry between the azido lysine 57 and the alkyne group of the Pentynoic-(PEG)₂-p6, **pentynoic-(PEG)₂-p6 (1.5 eq) and TFF3(36-59)(C57AzK)** (1.0 eq) were dissolved in a reaction buffer (10 mM NH₄HCO₃) in a 1.5 mL Eppendorf tube. Subsequently, aminoguanidine (100 eq) was added from a stock 200 mM solution. Then, the mixture of CuSO₄ and THPTA was added to the reaction mixture. Finally, sodium ascorbate (100 eq) was added to the reaction and the pH was adjusted to 8.0 by HCl/KOH. Nitrogen was let to flow into the reaction tube. The reaction tube was then immediately sealed. Incubation followed for 12 h, with stirring at 37 °C. Controls were made by dissolving 1 µL of reaction crude in 20 µL 30% MeCN/H₂O, and then adding tris(2-carboxyethyl)phosphine (TCEP) to reach a 30 mM concentration. After incubation at 37°C for 5 minutes, the samples were analyzed by HPLC-mass spectrometry (HPLC-MS).

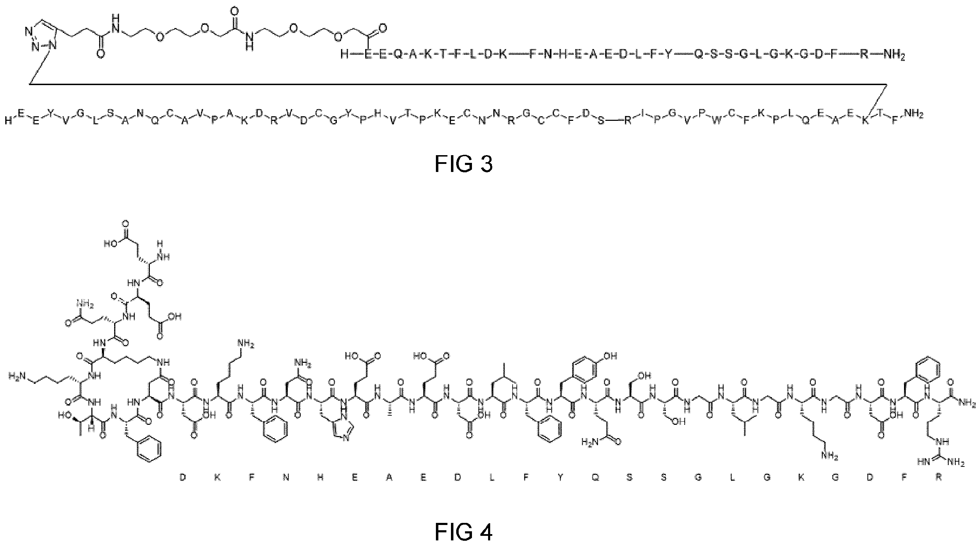

For **native chemical ligation between TFF3(1-35)-Nbz and TFF3(36-59)(C57K-Pentyp6),** TFF3(1-35)-Nbz (1.0 eq) and TFF3(36-59)(C57K-Pentyp6) (0.8 eq) were dissolved in reaction buffer (6M guanidinium hydrochloride, 100 mM of Na₂HPO₄, and 30 mM TCEP, the pH was adjusted to 7.0 with HCl/NaOH). Then2-mercaptoethanesulfonate (MeSNa) (100 eq) was added, and the reaction was kept on stirring for 4h at room temperature. Controls were made by dissolving 1 µL of reaction crude in 20 µL 30% acetonitrile (MeCN)/H₂O, and then adding TCEP to reach a 30 mM concentration. After incubation at 37 °C for 5 minutes, the samples were analyzed by HPLC-MS. When no more trace of the thioester were left, the volume of the reaction was increased to 3 mL by adding 30% MeCN/H₂O with 0.1% TFA, filtered, and injected on a preparative HPLC system for purification. **TFF3-C57K(Penty-p6)** was obtained, having a [M+H]⁺ = 10600.96 g/mol, [M+8H]⁸⁺/8 = 1326.1 g/mol, [M+9H]⁹⁺/9 = 1178.8 g/mol [M+10H]¹⁰⁺/10 = 1061.1 g/mol. The primary structure is illustrated in Figure 3.

**Native TFF3** was also produced in a recombinant way. To achieve this, the protocol of Järvå et al., 2020 was followed and adjusted.

The TFF3 sequence was then extended at the 5' end to introduce purification tags. Afterwards, the product was integrated into the plasmid pET28b for bacterial expression using sequence- and ligation-independent cloning (SLIC). The final plasmid was approved by sequencing and transformed into BL21CodonPlus(DE3) RIPL (R = Arginine, I = Isoleucine Ile, P = Proline, L = Leucine) cells for bacterial expression. This will be explained in the following in more detail.

### Preparation of Exponential Growth Stock-Culture of Transformed E. coli BL21CodonPlus(DE3) RIPL

*E. coli* BL21CodonPlus(DE3) RIPL cells were transformed with the according plasmid. The cells were streaked out on lysogeny broth (LB) agar plates containing 50 µg/mL Streptomycin, 25 µg/µL Chloramphenicol and 50 µg/mL Kanamycin and incubated in an incubator at 37 °C over night. One colony was picked and a LB medium containing 50 µg/mL Streptomycin, 25 µg/µL Chloramphenicol and 50 µg/mL Kanamycin was inoculated and incubated in a rotary shaker incubator at 250 rpm, at 37 °C over night. Terrific broth (TB) medium was inoculated with the LB culture with a starting optical density measured at 600 nm (OD₆₀₀) of 0.02 and incubated in a rotary shaker incubator at 250 rpm and 37 °C. The culture was grown to an OD₆₀₀ of >2.867. 7.527% DMSO were added to this culture, and the mixture (pre-culture) was snap-frozen in liquid nitrogen and stored at -80 °C for further use.

### Expression and Washing of Proteins

TB medium was inoculated with an OD₆₀₀ of 0.04 with the pre-culture. The culture was incubated in a rotary shaker incubator at 250 rpm, 26 °C for 24 hours. After 24 hours, the bacterial culture was cooled down on ice for 15 minutes and spun down with a centrifuge at 3000 g for 15 minutes. The supernatant was discarded and the cells were resuspended in washing buffer (HEPES based, pH 7) + DNase I + phenylmethylsulfonylfluorid (PMSF). The suspension was spun down at 3000 g for 15 min and the supernatant was discarded. The washing step was repeated.

The target proteins were purified upon tandem affinity purification with His-tag, and Strep-tag (binding motif for streptavidin) purification. Protease cleavage sites allow the elution of proteins from the columns. The eluted protein was dialyzed and verified upon SDS-PAGE.

### Manufacturing of lactam-stapled peptides

All lactam-stapled peptides were made on Rink-amide resin. The building blocks for the isopeptide bond formation were N-α-Fmoc-N-ε-4-methyltrityl-L-lysine (hereinafter denoted as MttK) and N-α-Fmoc-L-aspartic acid β-2-phenylisopropyl ester (hereinafter denoted as PhiPr). After SPPS, the peptide bound in the resin was treated with 2 % TFA in dichloromethane (DCM) to induce 4-methyltrityl and 2- phenylisopropyl deprotection. Then, isopeptide bond formation was allowed to take place, followed by adding 0.9 equivalents of HATU and 2 equivalents DIPEA, in DMF. After extensive rinsing in DMF and DCM, a second addition of coupling cocktail was done. The peptides were cleaved from the resin using 95:2.5:2.5 of TFA:H₂O:TIPS and precipitated with cold ether. The peptides were then filtered, dried, and redissolved in 30 % MeCN/H2O with 0.1 % TFA to a final volume of 3 mL. After filtration, the samples were injected in a preparative HPLC system for purification.

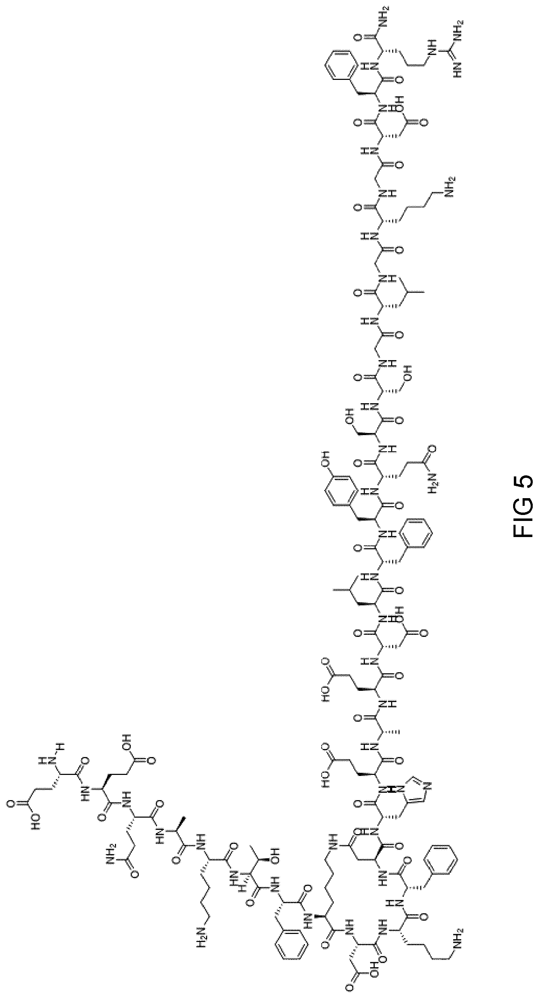

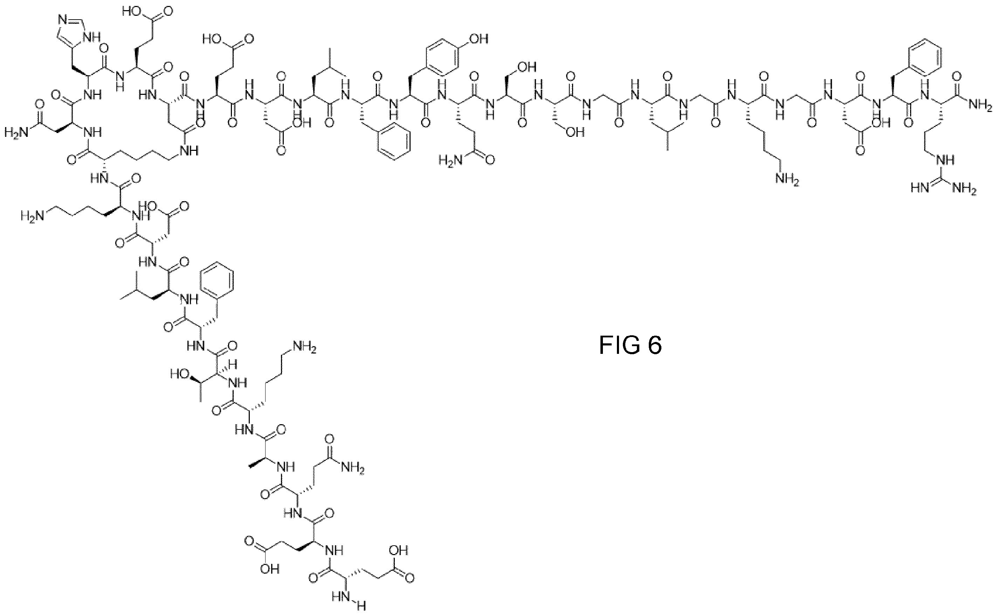

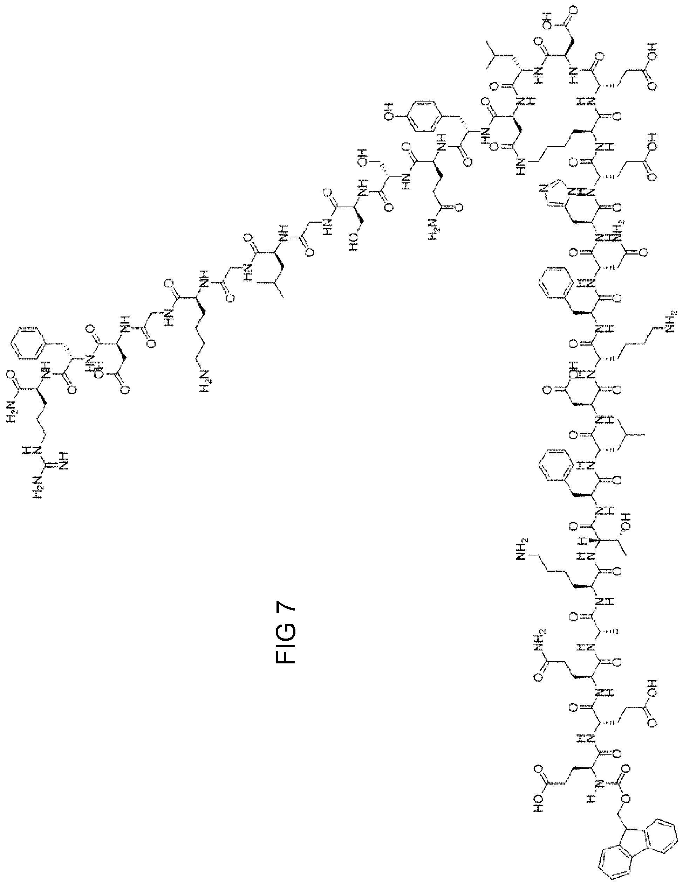

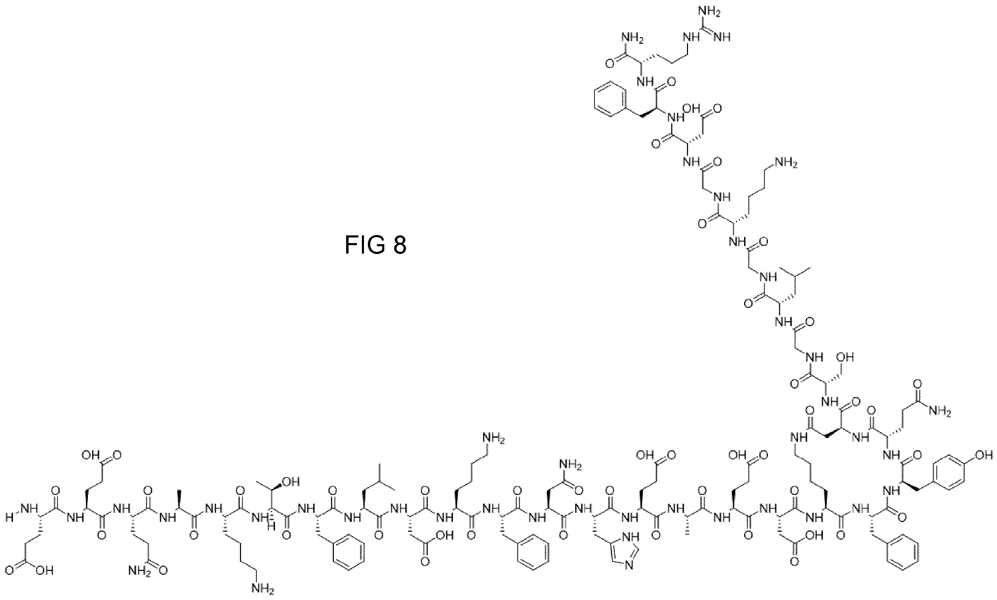

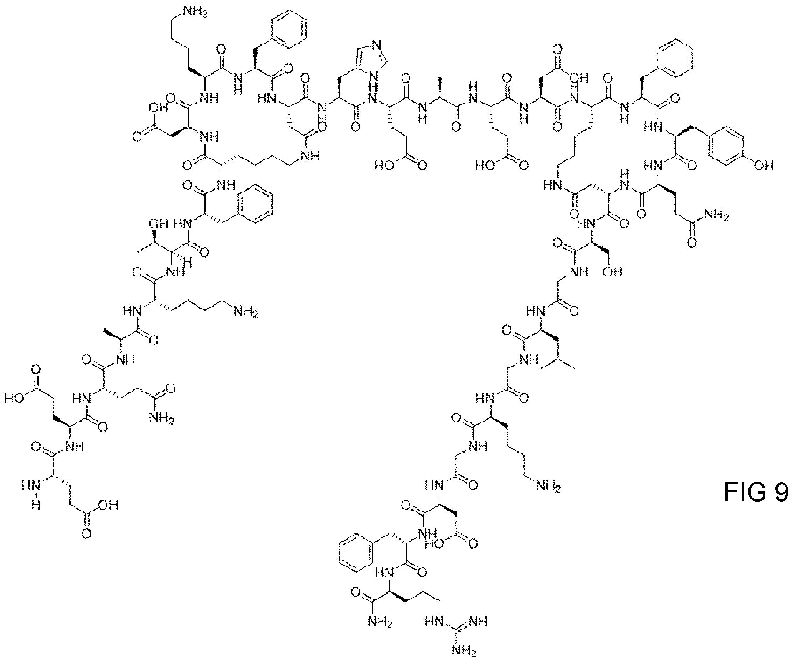

The resulting structures of the lactam-stapled peptides are illustrated in Figures 4 to 9.

The binding affinity of different cyclic virus binding moieties towards SARS-CoV-2 (expressed as the dissociation constant Kd) is summarized in the following Table 2. It was determined by microscale thermophoresis (MST). It can be well seen that the cyclic p6 peptides bind to SARS-CoV-2 with micromolar binding constants.

**Table 2: Properties of different cyclic virus binding moieties.**

| **Peptide name** | **Sequence** | **SEQ ID NO.** | **Substitutions** | **Helicity / %** | **Kd** / **µM** |
|---|---|---|---|---|---|
| p6(K)₄(D)₈ | EEQ(K4)KTF(D8)DKFN HEAEDLFYQSSGLGK GDFR | 4 | A4K, L8D | 2 | n.d. |
| p6(K)₈(D)₁₂ | EEQAKTF(K8)DKF(D12) HEAEDLFYQSSGLGK GDFR | 5 | L8K, N12D | 10 | 5 ± 3 |
| p6(K)₁₁(D)₁₅ | EEQAKTFLDK(K11)NH E(D15)EDLFYQSSGLG KGDFR | 6 | F11K, A15D | n.d. | n.d. |
| p6(K)₁₅(D)₁₉ | EEQAKTFLDKFNHE (K15)EDL(D19)YQSSGL GKGDFR | 7 | A15K, F19D | n.d. | n.d. |
| P6(K)₁₈(D)₂₂ | EEQAKTFLDKFNHEA ED(K18)FYQ(D22)SGLG KGDFR | 8 | L18K, S22D | 8 | n.d. |
| p6(K)_{8,18}(D)_{12,22} | EEQAKTF(K8)DKF(D12) HEAED(K18)FYQ(D22)S GLGKGDFR | 9 | A4K, L8D, L18K, S22D | 28 | 10 ± 1 |

| | | | | | |
|---|---|---|---|---|---|
| n.d. = not determined | | | | | |

### Manufacturing of stapled virus binding moieties by ring closing metatesis (RCM)

For RCM reactions, the following non-canonical amino acids were used:
S5Fmoc = Fmoc-(S)-2-(4-pentenyl)Ala-OH
R8Fmoc = Fmoc-(R)-2-(7-octenyl)Ala-OH

For these amino acids (AA) the coupling conditions were: 3.0 equivalents AA, 2.9 eq HATU, 6 eq DIPEA. DMF was added to reach a 0.2 M concentration with respect to the AA.

For all natural amino acids, the coupling conditions were: 5.0 equivalents AA, 4.9 eq HATU, 10 eq DIPEA. DMF was added to reach a 0.2 M concentration with respect to the AA.

After having confirmed the mass of the non-stapled peptide by test cleavage, RCM was performed. The resin was washed with 1 ml of DCM (3 × 1 min) and then with 1 ml of 1,2-Dichloroethane (DCE) (3 × 1 min). The resin (50 µmol) was treated with 1 ml of a 6 mM solution of Grubbs first-generation catalyst in DCE (20 mol% with respect to the resin substitution). The resulting suspension was agitated with continuous bubbling under nitrogen at 20-25 °C for 2 h. Afterwards, the solvent was drained. A double addition of Grubbs catalyst was then performed. Subsequently, the resin was washed with 1 ml of DCE (3 × 1 min) and then with 1 ml of DCM (3 × 1 min) and then dried under a stream of nitrogen. Peptide cleavage from the resin was done using 95:2.5:2.5 of TFA:H₂O:TIPS. The crude was precipitated with cold ether, filtered, and dried. The product was redissolved in 30% MeCN/H2O with 0.1 % TFA to a final volume of 3 mL. After filtration, the samples was injected in a preparative HPLC system for purification.

Acetylation was performed on the RCM products by swelling the Fmoc-deprotected resin in 1 ml of N-methyl-2-pyrrolidone (NMP) for 10 min and draining the solvent. Then, 2 ml of a solution of acetic anhydride and DIPEA in NMP (85:315:1,600 in volume) was prepared and added to the resin. Gently agitation of the mixture with bubbling under nitrogen was performed for 45 min and then the solvent was drained. Extensive washings followed with DCM and DMF. Finally, cleavage and purification was performed as outlined above.

After deprotection, the peptides contained i) an S5 residue (an alkenylated S-alanine) and an R8 residue (an alkenylated R-alanine) or ii) two S5 residues. An alkenyl bridge was formed between i) an S5 residue and an R8 residue or ii) between two S5 residues. This alkenyl bridge is a 7-undecenyl residue in case of an S5 residue and an R8 residue. Furthermore, the alkenyl bridge is a 4-octenyl residue in case of two S5 residues.

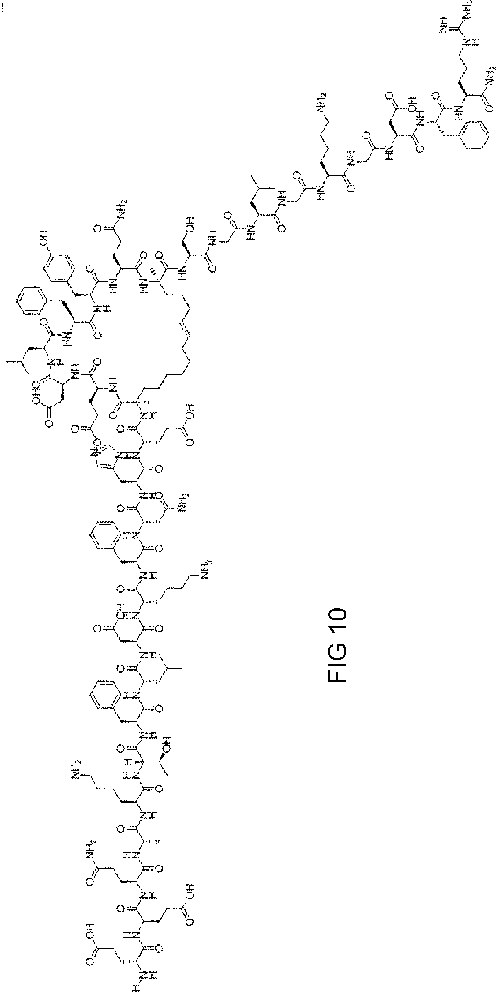

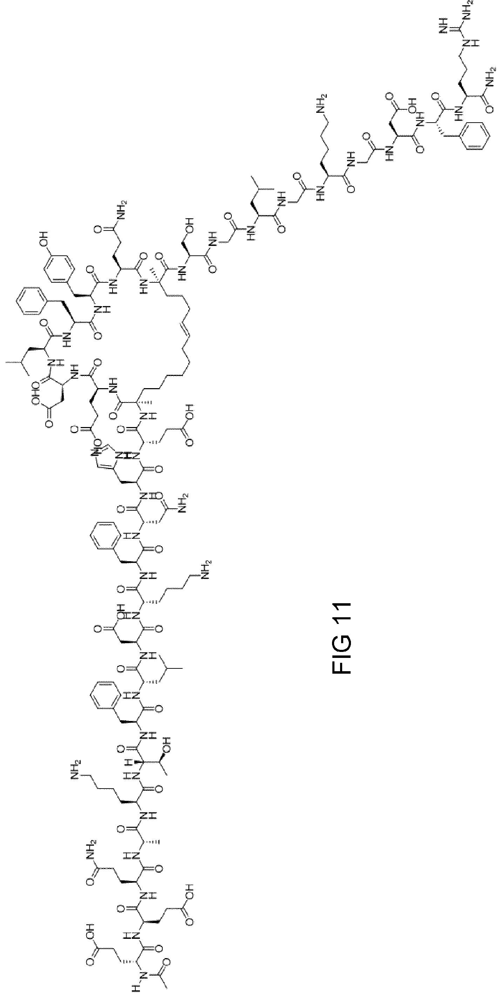

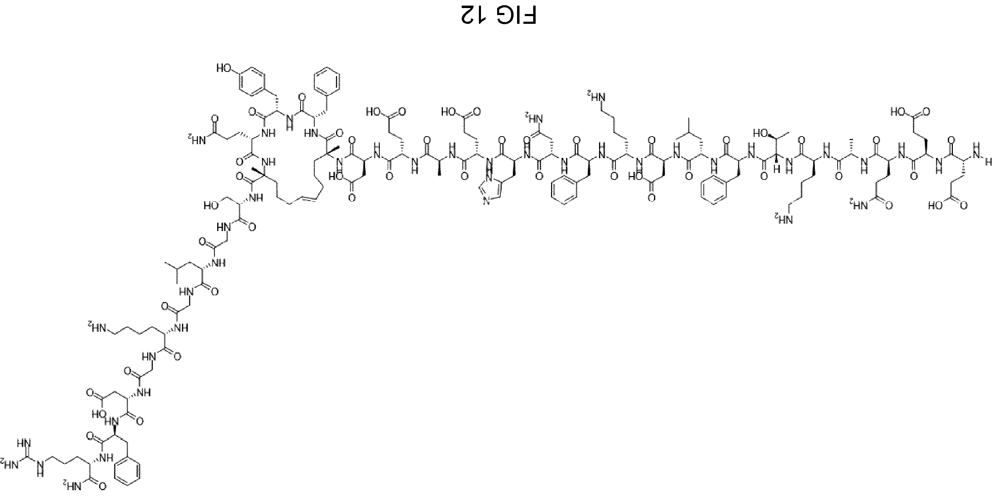

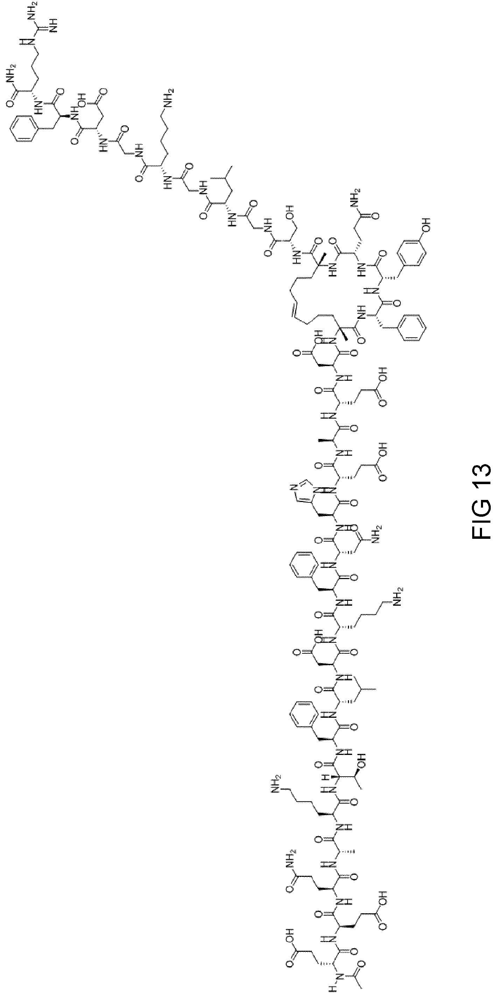

The resulting structures of the RCM-produced stapled peptides are illustrated in Figures 10 to 13.

The properties of different cyclic virus binding moieties including the binding affinity towards SARS-CoV-2 (expressed as the dissociation constant Kd) are summarized in the following Table 3. The binding affinity was determined by microscale thermophoresis (MST). It can be well seen that the cyclic p6 peptide p6(R8)₁₅(S5)₂₂ binds to SARS-CoV-2 with a nanomolar binding constant.

**Table 3: Properties of different cyclic virus binding moieties.**

| **Peptide name** | **Sequence** | **SEQ ID NO.** | **Substitutions** | **Helicity / %** | **Kd / µM** |
|---|---|---|---|---|---|
| p6(R8)₁₅(S5)₂₂ | EEQAKTFLDKFNHE (**R8**)EDLFYQ(**S5**)SGLG KGDFR | 10 | A15(R8), S22(S5) | 28 | 0.275 ± 0.142 |
| Ac-p6(R8)₁₅(S5)₂₂ | Ac-EEQAKTFLDKFNHE (**R8**)EDLFYQ(**S5**)SGLG KGDFR | 11 | A15(R8), S22(S5) | 25 | n.d |
| p6(S5)₁₈(S5)₂₂ | EEQAKTFLDKFNHEA ED(**S5**)FYQ(**S5**)SGLG KGDFR | 12 | L18(S5), S22(S5) | 4 | n.d. |
| Ac-p6(S5)₁₈(S5)₂₂ | Ac-EEQAKTFLDKFNHEA ED(**S5**)FYQ(**S5**)SGLG KGDFR | 13 | L18(S5), S22(S5) | 10 | n.d. |

| | | | | | |
|---|---|---|---|---|---|
| n.d. = not determined | | | | | |

### Peptide binding affinity measurements

Affinity measurements were performed using microscale thermophoresis (MST). Here, the ratio of chemical and thermal diffusion (Soret effect) impacts the mobility of a labelled compound (here: RBD) depending on the number of bound ligands (here: peptides). In MST, an infrared laser induces a local heat spot and subsequently a temperature gradient. Molecules move along this gradient depending on their characteristics at the water-protein interface. A bound ligand leads to changes of the ordered water shell at the observed protein, and may change or add also the effective surface potential. This leads to changes in the thermophoretic behavior indicated by an either increasing or decreasing thermophoresis amplitude. Upon the addition of different ligand concentrations, the change in fluorescence can be plotted (y-axis) against the ligand concentration. From the half-maximum change in fluorescence (inflexion point from saturation curve), the Kd of the protein-ligand interaction can be derived upon application of the mass-action law (Jerabek-Willemsen et al. 2014).

RBD labeling and purification was performed using a 2^{nd} generation NHS-Red labeling kit (NanoTemper). RBD solution was diluted with water to 10 µM, rebuffered in sodium carbonate aqueous solution at pH 8.0, and incubated in the dark at 300 µM of the red dye. Size exclusion chromatography allowed obtaining the labeled RBD free of unreacted dye. The labeling efficiency was analyzed from spectroscopy measurements to be approximately 1:1 (protein:dye). Affinity measurements were conducted in Dulbecco's Phosphate Buffered Saline (DPBS) (without Ca²⁺ and Mg²⁺) supplemented with 0.05 % (v/v) Tween20, and premium capillaries (NanoTemper).

MST was then measured by making 16 sequential 1:1 dilutions of each peptide, using PBS + 0.05 % Tween 20 as diluent, each with a final volume of 10 µL. Each diluted sample was then incubated with 10 µL of the 10 nM labeled RBD stock solution, therefore always keeping a 5 nM concentration of the labeled target protein in every sample.

MST signal was then acquired in a NanoTemper Technologies Monolith NT.115 Pico instrument, at an excitation power of 20% and a MST power of 40%. The signal was analyzed 1.5 seconds after the start of the IR-laser, and the obtained data was fitted as shown previously (Bhatia et al. 2017). These conditions were kept the same for all the measured samples.

### Affinity measurement of p6 versus RBD (SARS-CoV-2, Omicron B.1.1.529)

Additionally, chemically synthesized p6 peptide (according to SEQ ID NO. 3) was titrated against a constant concentration (5 nM) of fluorescently labelled RBD of SARS-CoV-2 omicron variant (strain B.1.1.529). A dissociation constant (Kd) of 280 nM was determined. Thus, the p6 peptide shows also a high binding affinity towards the omicron variant of SARS-CoV-2.

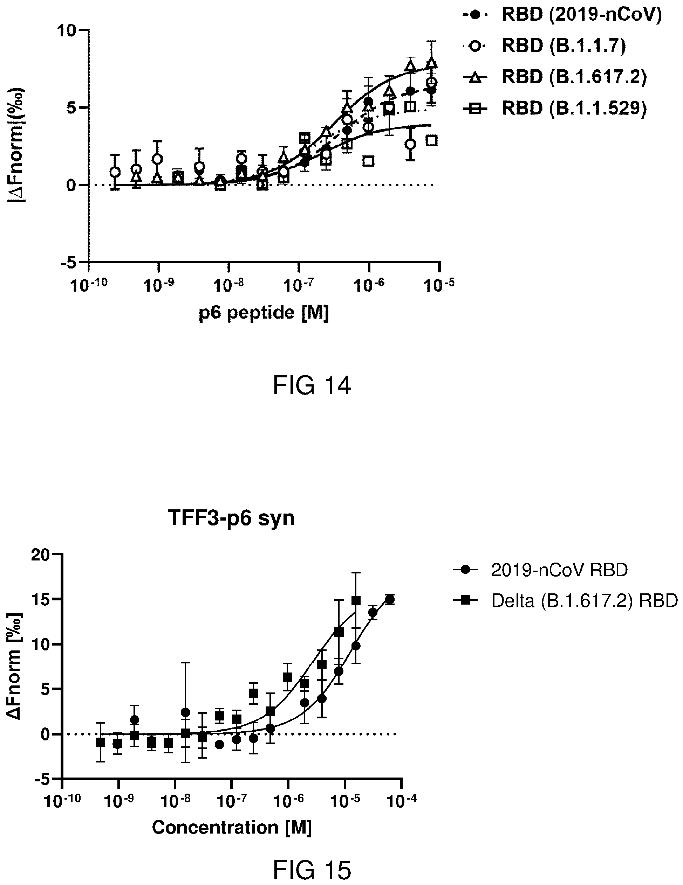

Figure 14 shows the results of the previously explained affinity characterization of the p6 peptide (according to SEQ ID NO. 3) using the MST technique against fluorescently labeled receptor binding domains (RBD) from wildtype (filled circles), alpha variant (unfilled circles), delta variant (triangles), and omicron variant (squares) of SARS-CoV-2 (wildtype, alpha variant and delta variant: N=3, error bars show SEM). It can be well seen that the p6 peptide binds to all SARS-CoV-2 variants/strains with high affinity (see also Table 1 for quantitative results of these experiments).

### p4 and p6 derivatives

A plurality of additional p4 and p6 derivatives was synthesized and the affinity of these p4 and p6 derivatives against the receptor binding domain was tested in MST experiments as explained above for the p6 peptide.

All p4 derivatives were synthesized as described in Sarto et al. 2022. The p6 derivatives p6-rigid, p6-dis and p6-rigid-dis were ordered from PSL (Peptide Specialty Laboratories GmbH, Heidelberg) as TFA-salt. Upon arrival, the peptides were reconstituted in PBS, snap-frozen in liquid nitrogen and stored at -80°C until further usage.

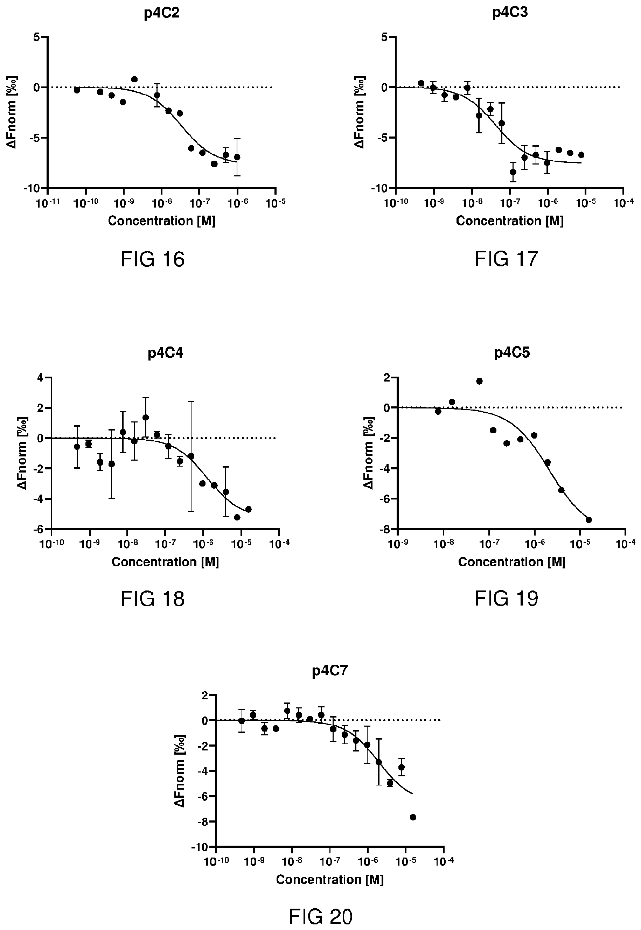

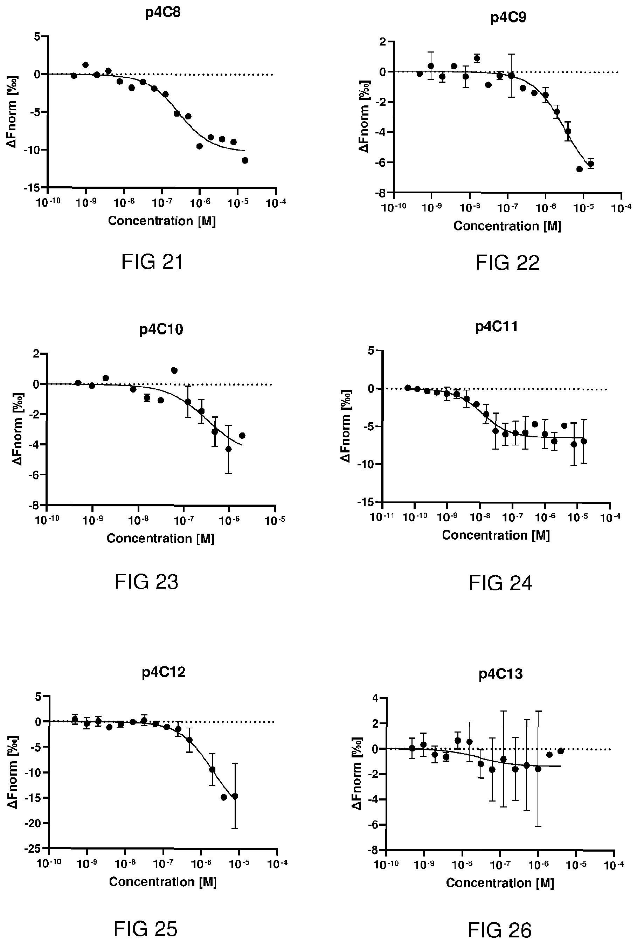

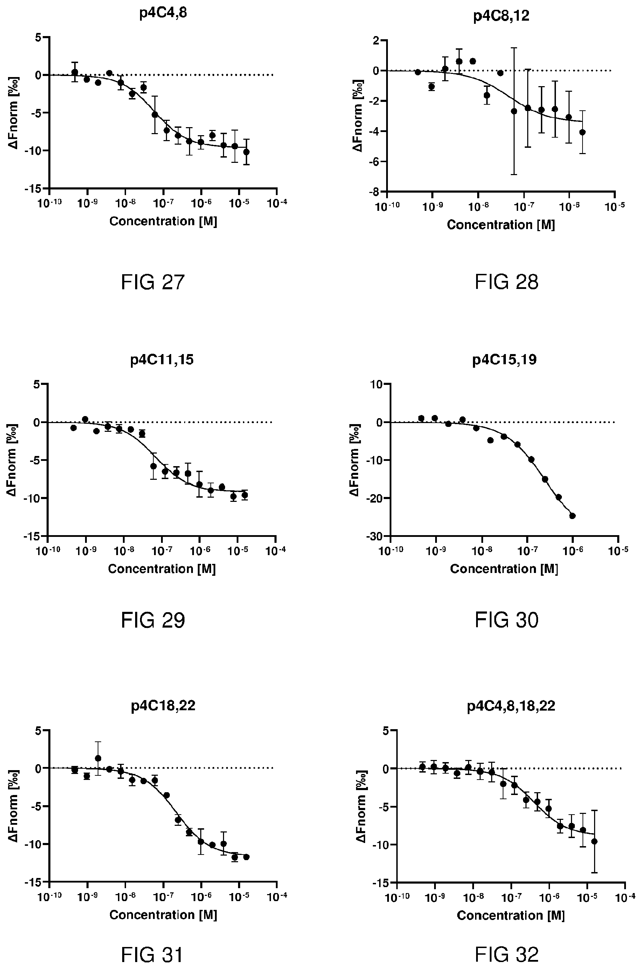

Table 4 lists the different tested p4 derivatives as well as the dissociation constants obtained from the affinity measurements. The cysteine residues of all peptides are in their normal, reduced state. The results of the affinity measurements are also illustrated in Figures 16 to 32.

**Table 4: Properties of p4 derivatives (virus binding moieties).**

| **Peptide name** | **Sequence** | **SEQ ID NO.** | **SARS-CoV-2 target strain** | **Kd/µM** | **95 % CI** / **µM^{*4}** |
|---|---|---|---|---|---|
| p4C2 | ECQAKTFLDKFNHEAEDLFYQSS | 23 | 2019-nCov | 0.034 | [0.02, 0.056] |
| p4C3 | EECAKTFLDKFNHEAEDLFYQSS | 24 | 2019-nCov | 0.042 | [0.024, 0.071] |
| p4C4 | EEQCKTFLDKFNHEAEDLFYQSS | 25 | 2019-nCov | 1.298 | [0.31, 6.241] |
| p4C5 | EEQACTFLDKFNHEAEDLFYQSS | 26 | 2019-nCov | 2.141 | [0.557, 7.241] |
| p4C6 | EEQAKCFLDKFNHEAEDLFYQSS | 27 | 2019-nCov | n.d. | n.d. |
| p4C7 | EEQAKTCLDKFNHEAEDLFYQSS | 28 | 2019-nCov | 1.941 | [0.791, 5.117] |
| p4C8 | EEQAKTFCDKFNHEAEDLFYQSS | 29 | 2019-nCov | 0.281 | [0.179, 0.432] |
| p4C9 | EEQAKTFLCKFNHEAEDLFYQSS | 30 | 2019-nCov | 3.226 | [1.901, 5.501] |
| p4C10 | EEQAKTFLDCFNHEAEDLFYQSS | 31 | 2019-nCov | 0.331 | [0.11, 0.958] |
| p4C 11 | EEQAKTFLDKCNHEAEDLFYQSS | 32 | 2019-nCov | 0.012 | [0.006, 0.021] |
| p4C12 | EEQAKTFLDKFCHEAEDLFYQSS | 33 | 2019-nCov | 2.048 | [1.026, 4.409] |
| p4C13 | EEQAKTFLDKFNCEAEDLFYQSS | 34 | 2019-nCov | 0.028 | [-0.012, 2.55] |
| p4C4,8 | EEQCKTFCDKFNHEAEDLFYQSS | 35 | 2019-nCov | 0.059 | [0.04, 0.085] |
| p4C8,12 | EEQAKTFCDKFCHEAEDLFYQSS | 36 | 2019-nCov | 0.047 | [0.007, 0.348] |
| p4C11,15 | EEQAKTFLDKCNHECEDLFYQSS | 37 | 2019-nCov | 0.068 | [0.047, 0.099] |
| p4C15,19 | EEQAKTFLDKFNHECEDLCYQSS | 38 | 2019-nCov | 0.238 | [0.155, 0.371] |
| p4C18,22 | EEQAKTFLDKFNHEAEDCFYQCS | 39 | 2019-nCov | 0.218 | [0.156, 0.302] |
| p4C4,8,18,22 | EEQCKTFCDKFNHEAEDCFYQCS | 40 | 2019-nCov | 0.400 | [0.156, 0.302] |

| | | | | | |
|---|---|---|---|---|---|
| ^{*4} CI = confidence interval n.d. = not determined | | | | | |

All p4 derivatives showing a dissociation constant against the SARS-CoV-2 RBD of less than 0.3, in particular less than 0.2, in particular less than 0.1, in particular less than 0.05, in particular less than 0.04, in particular less than 0.03, in particular less than 0.02, are particular appropriate virus binding moieties.

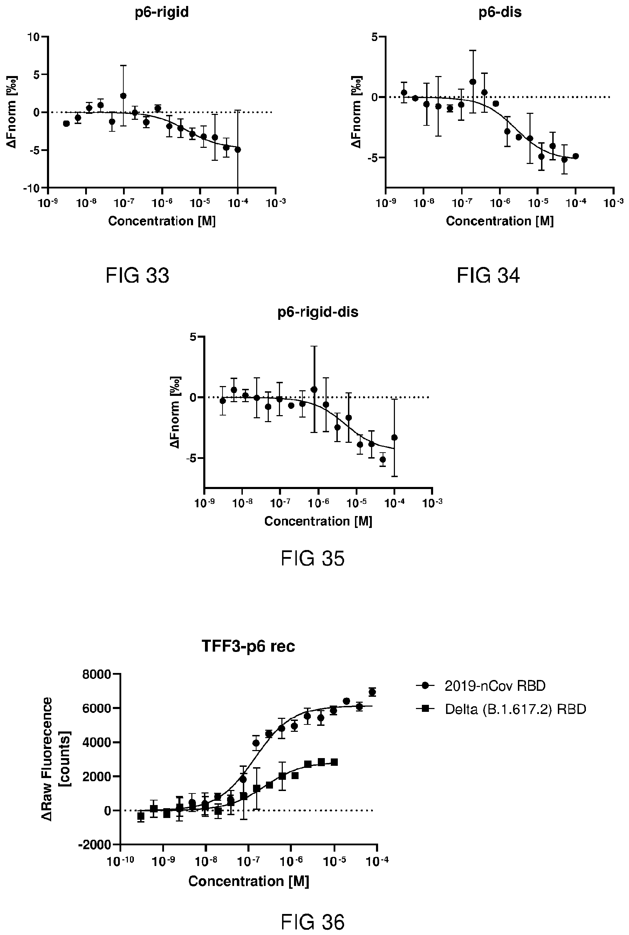

Table 5 lists the different tested p6 derivatives as well as the dissociation constants obtained from the affinity measurements. The cysteine residues of all peptides are in their oxidized state so that they form an intramolecular disulfide bond. The results of the affinity measurements are also illustrated in Figures 33 to 35.

**Table 5: Properties of p6 derivatives (virus binding moieties).**

| **Peptide name** | **Sequence** | **SEQ ID NO.** | **SARS-CoV-2 target strain** | **Kd/µM** | **95 % CI / µM** |
|---|---|---|---|---|---|
| p6-rigid | EAAAKEEQAKTFLDKFNHEAE DLFYQSSGLGKGDFR | 41 | 2019-nCov | 4.531 | [0.954, 21.4] |
| p6-dis | EEQAKTFLDKFNHEAEDLFYQ SCGLGKGDFRC | 42 | 2019-nCov | 2.596 | [1.174, 5.771] |
| p6-rigid-dis | EAAAKEEQAKTFLDKFNHEAE DLFYQSCGLGKGDFRC | 43 | 2019-nCov | 5.135 | [1.487, 16.9] |

### Recombinant production of TFF3-p6

TFF3-p6 was also produced in a recombinant way. To achieve this, the protocol of Järvå et al., 2020 was followed and adjusted.

The TFF3 sequence was then extended to introduce purification tags and a TEV (Tobacco Etch Virus) protease cleavage site at the 5' end and p6 at the 3' end. Afterwards, the product was integrated into the plasmid pET28b for bacterial expression using sequence- and ligation-independent cloning (SLIC). The final plasmid was approved by sequencing and transformed into BL21CodonPlus(DE3) RIPL cells for bacterial expression. This was done as explained above in detail in the paragraph "Preparation of Exponential Growth Stock-Culture of Transformed *E. coli* BL21CodonPlus(DE3) RIPL".

### Expression and Washing of Proteins

TB medium was inoculated with an OD₆₀₀ of 0.04 with the pre-culture. The culture was incubated in a rotary shaker incubator at 250 rpm, 26 °C for 24 hours. After 24 hours, the bacterial culture was cooled down on ice for 15 minutes and spun down with a centrifuge at 3000 g for 15 minutes. The supernatant was discarded and the cells were resuspended in washing buffer (HEPES based, pH 8). The suspension was spun down at 3000 g for 15 min and the supernatant was discarded. The washing step was repeated. The bacteria cells were lysed in lysis buffer (HEPES based, pH 8) by sonication. The lysate was spun down at 12.000 g for 45 minutes, the pellet discarded, and the supernatant filtered through a 0.2 µm filter.

### Purification of Proteins

The target proteins were purified upon tandem affinity purification with His-tag, and Strep-tag (binding motif for streptavidin) purification. Imidazole (ImH) was added to the filtered bacterial lysate to a final concentration of 40 mM. The lysate was applied to a HisTrap column (Cytiva) using an Äkta Pure 25 (Cytiva) fast protein liquid chromatography (FPLC) device. The column was washed with wash buffer (300 mM NaCl, 50 mM Tris-base, 40 mM ImH, pH 7.5), the proteins eluted with elution buffer (300 mM NaCl, 50 mM Tris-base, 400 mM ImH, pH 7.5) and the elute collected in fractions. Fractions containing the product of interest were identified via SDS-PAGE and pooled.

The pooled fractions were applied to a Strep-Tactin XT 4Flow column (IBA Lifesciences) using an Äkta Pure 25. The column was washed with wash buffer (100 mM Tris-HCl, 150 mM NaCl, 1 mM ETDA, pH 8), the proteins eluted with elution buffer (100 mM Tris-HCl, 150 mM NaCl, 1 mM ETDA, 50 mM biotin, pH 8) and the elute collected in fractions. Fractions containing the product of interest were identified via SDS-PAGE and pooled prior to dialysis against PBS (pH 7.4). The protein content was determined via the absorption spectrum at 280 nM using a NanoDrop2000 spectrophotometer (Thermo Fisher Scientific).

To remove the tandem affinity purification tags, the purified products were incubated over night at 30 °C with ProTEV Plus (Promega) according to the manufacturer's recommendations with 1 U ProTEV Plus per 15 µg of purified product.

ImH was added to the reaction mix to a final concentration of 40 mM before applying it to a HisTrap column using an Äkta Pure 25 and washing the column with wash buffer (300 mM NaCl, 50 mM Tris-base, 40 mM ImH, pH 7.5) The flow through with the final product (TFF3-p6) was collected in fractions, while the cleaved purification tags and the ProTEV Plus (also containing a His-tag) remained in the column. Fractions containing the final product were identified via SDS-PAGE, pooled and dialyzed against PBS (pH 7.4). The volume was reduced using a Vivaspin PES Centrifugal Concentrator (Sartorius) to a final protein concentration between 1 mg/ml and 3 mg/ml as determined by NanoDrop2000. The solution was snap-frozen in liquid nitrogen and stored at -20 °C until further usage. The correct final product and the purity was controlled by SDS-PAGE and Ultra-Performance-Liquid-Chromatography-Mass-Spectrometry (UPLC/MS).

### Affinity measurement of TFF-p6 versus RBD of SARS-CoV-2

In these experiments, chemically synthesized TFF3-p6 (TFF3-C57K(Penty-p6) or TFF3-p6 syn; the primary structure of which is illustrated in Figure 3 and reproduced in SEQ ID NO. 18) and recombinant TFF3-p6 (TFF3-p6 rec) according to SEQ ID NO. 19 were titrated against a constant concentration (5 nM) fluorescently labelled RBD of the wildtype SARS-CoV2 (also denoted as nCov2019) and o the Delta variant of SARS-CoV2 (also denoted as Delta (B.1.617.2)). The resulting dissociation constants (Kd) are listed in Table 6. The results of this affinity measurement are shown in Figure 15 for TFF3-p6 syn and in Figure 36 for TFF3-p6 rec.

**Table 6: Dissociation constants of affinity measurements of TFF-p6 versus RBD of SARS-CoV-2.**

| **Peptide name** | **SEQ ID NO.** | **SARS-CoV-2 taget strain** | **Kd /µM** | **95% CI /µM** |
|---|---|---|---|---|
| TFF3-p6 rec | 19 | 2019-nCov | 0.141 | [0.109, 0.182] |
| TFF3-p6 rec | 19 | Delta (B.1.617.2) | 0.243 | [0.117, 0.495] |
| TFF3-p6 syn | 18 | 2019-nCov | 12.69 | [6.87, 23.8] |
| TFF3-p6 syn | 18 | Delta (B.1.617.2) | 2.672 | [1.263, 5.67] |

### Cytotoxicity Assay

Cell viability was determined using a Cell Counting Kit (Hycultec, HY-K0301) according to the manufacturer's instructions.

A549, HBE and Calu-3 cells were cultured in DMEM supplemented with 10% (v/v) FBS, 100 U/mL penicillin and 100 µg/mL streptomycin. Cells were seeded in a 96-well plate at a density of 5 x 10⁴ cells/mL in 90µl DMEM Medium per well over night at 37°C and 5% CO₂.

10 µl of recombinant TFF3-p6 or tags-TFF3-p6 (still containing the affinity tags) solved in deionized water were added in serial dilutions (6.6 x 10⁻⁶ M - 6.6 x 10⁻¹⁰ M) including positive (1% SDS) and negative controls (vehicle; medium, H₂O) and incubated for another 48 h at 37°C and 5 % CO₂. For background subtraction, also wells containing no cells but only sample were used. After 48 h incubation the CCK8 solution was added (10 µl/well) and absorbance (450 nm/650 nm) was measured after approximately 3h incubation of the dye using a Tecan plate reader (SPARK, TECAN-reader Tecan Group Ltd.) The cell viability was calculated by setting the non-treated control to 100% after subtracting the background signal.

Neither recombinant TFF3-p6 nor tags-TFF3-p6 showed a significant difference to the negative controls. Rather, the cell viability was approximately 100 % in all cases. In contrast, the positive controls showed a cell viability of or close to 0 %.

### Antiviral Efficacy

To test the antiviral efficacy of TFF3-p6, a pseudoviral infection system was used. The system is based on a vesicular stomatitis virus (VSV) which lacks the VSV envelope glycoprotein but contains an eGFP and SARS-CoV-2 spike protein (VSVΔG-CoV-2; Markus Hoffmann et al.).

TFF3-p6 or PBS (control) was incubated with VSVΔG-CoV-2 for 30 min at a final concentration of 50 µM TFF3-p6 and 2.625 x 10⁴ ffu/mL virus in 100 µl. The mixture was added to VeroE6 cells and incubated for 24 h at 37 °C in a cell culture incubator. Pictures were taken using a fluorescence microscope and the number of infected cells was determined.

TFF3-p6 could reduce the infection of VeroE6 cells with VSVΔG-CoV2 pseudovirus by about 40 % as compared to the PBS control.

### List of references cited in the preceding sections

1. Bhatia, Sumati, et al. "Linear polysialoside outperforms dendritic analogs for inhibition of influenza virus infection in vitro and in vivo." Biomaterials 138 (2017): 22-34.
2. Braga Emidio, Nayara, et al. "Chemical Synthesis of TFF3 Reveals Novel Mechanistic Insights and a Gut-Stable Metabolite." Journal of Medicinal Chemistry 64.13 (2021): 9484-9495.
3. Bui, Vuong N., et al. "Pathogenicity of an H5N1 avian influenza virus isolated in Vietnam in 2012 and reliability of conjunctival samples for diagnosis of infection." Virus Research 179 (2014): 125-132.
4. Cabán, Madelyn, et al. "Cross-protective antibodies against common endemic respiratory viruses." Nature Communications 14.1 (2023): 798.
5. Han, Dong P., Adam Penn-Nicholson, and Michael W. Cho. "Identification of critical determinants on ACE2 for SARS-CoV entry and development of a potent entry inhibitor." Virology 350.1 (2006): 15-25.
6. Hoffmann, Markus, et al. "SARS-CoV-2 cell entry depends on ACE2 and TMPRSS2 and is blocked by a clinically proven protease inhibitor." Cell 181.2 (2020): 271-280.
7. Hoffmann, Miriam, Nicole L. Snyder, and Laura Hartmann. "Polymers Inspired by Heparin and Heparan Sulfate for Viral Targeting." Macromolecules 55.18 (2022): 7957-7973.
8. Hoffmann, Werner. "Trefoil factor family (TFF) peptides and their different roles in the mucosal innate immune defense and more: An update." *Current Medicinal Chemistry* 28.36 (2021): 7387-7399.
9. Hrebik, Dominik, et al. "ICAM-1 induced rearrangements of capsid and genome prime rhinovirus 14 for activation and uncoating." *Proceedings of the National Academy of Sciences* 118.19 (2021): e2024251118.
10. Issmail, Leila, et al. "Prefusion-specific antibody-derived peptides trivalently presented on DNA-nanoscaffolds as an innovative strategy against RSV entry." Frontiers in Virology 2 (2022).
11. Järvå, Michael A., et al. "Trefoil factors share a lectin activity that defines their role in mucus." Nature Communications 11.1 (2020): 1-9.
12. Jerabek-Willemsen M., et al. "MicroScale Thermophoresis: Interaction analysis and beyond." Journal of Molecular Structure 1077 (2014): 101-113.
13. Khan, Abdul Ghafoor, et al. "Human rhinovirus type 54 infection via heparan sulfate is less efficient and strictly dependent on low endosomal pH." Journal of Virology 81.9 (2007): 4625-4632.
14. Lauster, Daniel, et al. "Multivalent peptide-nanoparticle conjugates for influenza-virus inhibition." Angewandte Chemie International Edition 56.21 (2017): 5931-5936.
15. Madsen, Anders, et al. "Human antibodies targeting influenza B virus neuraminidase active site are broadly protective." Immunity 53.4 (2020): 852-863.
16. McLellan, Jason S., et al. "Structure-based design of a fusion glycoprotein vaccine for respiratory syncytial virus." science 342.6158 (2013): 592-598.
17. Memczak, Henry, et al. "Anti-hemagglutinin antibody derived lead peptides for inhibitors of influenza virus binding." PLoS One 11.7 (2016): e0159074.
18. Sarto, Carolina, et al. "Atomistic insight into the essential binding event of ACE2-derived peptides to the SARS-CoV-2 spike protein." Biological Chemistry 403.5-6 (2022): 615-624.
19. Smith, Thomas J., et al. "Neutralizing antibody to human rhinovirus 14 penetrates the receptor-binding canyon." Nature 383.6598 (1996): 350-354.
20. Thim, L., F. Madsen, and S. S. Poulsen. "Effect of trefoil factors on the viscoelastic properties of mucus gels." European Journal of Clinical Investigation 32.7 (2002): 519-527.
21. Zhu, Qing, et al. "A highly potent extended half-life antibody as a potential RSV vaccine surrogate for all infants." Science Translational Medicine 9.388 (2017): eaaj1928.
22. Zuo, Teng, et al. "Comprehensive analysis of antibody recognition in convalescent humans from highly pathogenic avian influenza H5N1 infection." Nature Communications 6.1 (2015): 8855.

## Claims

1. Peptide comprising a virus binding moiety (2) and a mucin binding moiety (1) covalently bound to the virus binding moiety (2),
**characterized**
**in that** the virus binding moiety (2) comprises or is a peptide being at least 95 % identical to SEQ ID NO. 48; and
**in that** the mucin binding moiety (1) comprises or is a peptide being at least 95 % identical to SEQ ID NO. 1, SEQ ID NO. 14, SEQ ID NO. 20, SEQ ID NO. 21, or SEQ ID NO. 22.

2. Peptide according to claim 1, **characterized in that** the mucin binding moiety (1) comprises or is a jacalin peptide being at least 95 % identical to SEQ ID NO. 22.

3. Peptide according to claim 1, **characterized in that** the mucin binding moiety (1) comprises or is a trefoil factor 3 peptide being at least 95 % identical to SEQ ID NO. 1.

4. Peptide according to claim 1, **characterized in that** the mucin binding moiety (1) comprises or is a trefoil factor 3 peptide being at least 95 % identical to SEQ ID NO. 14.

5. Peptide according to claim 1, **characterized in that** the mucin binding moiety (1) comprises or is a trefoil factor 1 peptide being at least 95 % identical to SEQ ID NO. 20.

6. Peptide according to claim 1, **characterized in that** the mucin binding moiety (1) comprises or is a trefoil factor 2 peptide being at least 95 % identical to SEQ ID NO. 21.

7. Peptide according to any of the preceding claims, **characterized in that** the peptide comprises at least two virus binding moieties (2), wherein the mucin binding moiety (1) is covalently bound to at least one of the at least two virus binding moieties (2).

8. Peptide according to any of the preceding claims for use in preventing or treating a viral infection.

9. Peptide according to any of claims 1 to 7 for use according to claim 8, **characterized in that** the viral infection is an infection with influenza A virus.

10. Peptide according to any of claims 1 to 7 for use according to claim 9, **characterized in that** the influenza A virus is a virus of strain H1, H3, or H7.

11. Medicament comprising a peptide according to any of claims 1 to 7 as pharmaceutically active ingredient.

12. Medicament according to claim 11, **characterized in that** the medicament is designed and configured to be administered as a nasal spray or as an inhalation spray.

13. Method for manufacturing a peptide according to any of claims 1 to 7, comprising the following steps:
a) reacting a virus binding moiety precursor with a first mucin binding moiety precursor, the first mucin binding moiety precursor comprising a first part of the mucin binding moiety (1), thus obtaining a peptide precursor; and
b) reacting the peptide precursor with a second mucin binding moiety precursor, the second mucin binding moiety precursor comprising a second part of the mucin binding moiety (1).

14. Method according to claim 13, **characterized in that** the virus binding moiety precursor comprises a 4-pentynoic moiety covalently bound to a virus binding moiety (2).

15. Method according to claim 13 or 14, **characterized in that** the first mucin binding moiety precursor comprises less than the half of all amino acids of the mucin binding moiety (1).

## Patentansprüche

1. Peptid mit einem virusbindenden Anteil (2) und einem mucinbindenden Anteil (1), der kovalent an den virusbindenden Anteil (2) gebunden ist,
**dadurch gekennzeichnet,**
**dass** der virusbindende Anteil (2) ein Peptid umfasst oder ist, das zu mindestens 95% mit SEQ ID NO. 48 identisch ist; und
**dass** der mucinbindende Anteil (1) ein Peptid umfasst oder ist, das zu mindestens 95% mit SEQ ID NO. 1, SEQ ID NO. 14, SEQ ID NO. 20, SEQ ID NO. 21 oder SEQ ID NO. 22 identisch ist.

2. Peptid nach Anspruch 1, **dadurch gekennzeichnet, dass** der mucinbindende Anteil (1) ein Jacalinpeptid umfasst oder ist, das zu mindestens 95% mit SEQ ID NO. 22 identisch ist.

3. Peptid nach Anspruch 1, **dadurch gekennzeichnet, dass** der mucinbindende Anteil (1) ein Trefoil-Faktor-3-Peptid umfasst oder ist, das zu mindestens 95% mit SEQ ID NO. 1 identisch ist.

4. Peptid nach Anspruch 1, **dadurch gekennzeichnet, dass** der mucinbindende Anteil (1) ein Trefoil-Faktor-3-Peptid umfasst oder ist, das zu mindestens 95% mit SEQ ID NO. 14 identisch ist.

5. Peptid nach Anspruch 1, **dadurch gekennzeichnet, dass** der mucinbindende Anteil (1) ein Trefoil-Faktor-1-Peptid umfasst oder ist, das zu mindestens 95% mit SEQ ID NO. 20 identisch ist.

6. Peptid nach Anspruch 1, **dadurch gekennzeichnet, dass** der mucinbindende Anteil (1) ein Trefoil-Faktor-2-Peptid umfasst oder ist, das zu mindestens 95% mit SEQ ID NO. 21 identisch ist.

7. Peptid nach einem der vorhergehenden Ansprüche, **dadurch gekennzeichnet, dass** das Peptid mindestens zwei virusbindende Anteile (2) umfasst, wobei der mucinbindende Anteil (1) kovalent an mindestens einen der mindestens zwei virusbindenden Anteile (2) gebunden ist.

8. Peptid nach einem der vorhergehenden Ansprüche zur Verwendung bei der Vorbeugung oder Behandlung einer Virusinfektion.

9. Peptid nach einem der Ansprüche 1 bis 7 zur Verwendung gemäß Anspruch 8, **dadurch gekennzeichnet, dass** die Virusinfektion eine Infektion mit dem Influenza-A-Virus ist.

10. Peptid nach einem der Ansprüche 1 bis 7 zur Verwendung gemäß Anspruch 9, **dadurch gekennzeichnet, dass** das Influenza-A-Virus ein Virus vom Stamm H1, H3 oder H7 ist.

11. Medikament mit einem Peptid nach einem der Ansprüche 1 bis 7 als pharmazeutisch wirksamer Bestandteil.

12. Medikament nach Anspruch 11, **dadurch gekennzeichnet, dass** das Medikament dazu ausgelegt und ausgebildet ist, als Nasenspray oder als Inhalationsspray verabreicht zu werden.

13. Verfahren zur Herstellung eines Peptids nach einem der Ansprüche 1 bis 7, mit den folgenden Schritten:
a) Reagieren eines Vorläufers eines virusbindenden Anteils mit einem ersten Vorläufer eines mucinbindenden Anteils, wobei der erste Vorläufer des mucinbindenden Anteils einen ersten Teil des mucinbindenden Anteils (1) umfasst, sodass ein Peptidvorläufer erhalten wird; und
b) Reagieren des Peptidvorläufers mit einem zweiten Vorläufer des mucinbindenden Anteils, wobei der zweite Vorläufer des mucinbindenden Anteils einen zweiten Teil des mucinbindenden Anteils (1) umfasst.

14. Verfahren nach Anspruch 13, **dadurch gekennzeichnet, dass** der Vorläufer des virusbindenden Anteils einen 4-Pentinsäure-Anteil umfasst, der kovalent an einen virusbindenden Anteil (2) gebunden ist.

15. Verfahren nach Anspruch 13 oder 14, **dadurch gekennzeichnet, dass** der erste Vorläufer des mucinbindenden Anteils weniger als die Hälfte aller Aminosäuren des mucinbindenden Anteils (1) umfasst.

## Revendications

1. Peptide comprenant une fraction de liaison au virus (2) et une fraction de liaison à la mucine (1) liée de manière covalente à la fraction de liaison au virus (2),
**caractérisé**
**en ce que** la fraction de liaison au virus (2) comprend ou est un peptide identique à au moins 95 % à SEQ ID NO. 48 ; et
**en ce que** la fraction de liaison à la mucine (1) comprend ou est un peptide identique à au moins 95 % à SEQ ID NO. 1, SEQ ID NO. 14, SEQ ID NO. 20, SEQ ID NO. 21 ou SEQ ID NO. 22.

2. Peptide selon la revendication 1, **caractérisé en ce que** la fraction de liaison à la mucine (1) comprend ou est un peptide de jacaline identique à au moins 95 % à SEQ ID NO. 22.

3. Peptide selon la revendication 1, **caractérisé en ce que** la fraction de liaison à la mucine (1) comprend ou est un peptide du facteur 3 de trefoil étant identique à au moins 95 % à SEQ ID NO. 1.

4. Peptide selon la revendication 1, **caractérisé en ce que** la fraction de liaison à la mucine (1) comprend ou est un peptide du facteur 3 de trefoil étant identique à au moins 95 % à SEQ ID NO. 14.

5. Peptide selon la revendication 1, **caractérisé en ce que** la fraction de liaison à la mucine (1) comprend ou est un peptide du facteur 1 de trefoil étant identique à au moins 95 % à SEQ ID NO. 20.

6. Peptide selon la revendication 1, **caractérisé en ce que** la fraction de liaison à la mucine (1) comprend ou est un peptide du facteur 2 de trefoil étant identique à au moins 95 % à SEQ ID NO. 21.

7. Peptide selon l'une quelconque des revendications précédentes, **caractérisé en ce que** le peptide comprend au moins deux fractions de liaison au virus (2), dans lequel la fraction de liaison à la mucine (1) est lié de manière covalente à au moins l'un des au moins deux fractions de liaison au virus (2).

8. Peptide selon l'une quelconque des revendications précédentes pour utilisation dans la prévention ou le traitement d'une infection virale.

9. Peptide selon l'une quelconque des revendications 1 à 7 pour utilisation selon la revendication 8, **caractérisé en ce que** l'infection virale est une infection par le virus de la grippe A.

10. Peptide selon l'une quelconque des revendications 1 à 7 pour utilisation selon la revendication 9, **caractérisé en ce que** le virus de la grippe A est un virus de souche H1, H3, ou H7.

11. Médicament comprenant un peptide selon l'une quelconque des revendications 1 à 7 comme ingrédient pharmaceutiquement actif.

12. Médicament selon la revendication 11, **caractérisé en ce que** le médicament est conçu et configuré pour être administré sous forme de spray nasal ou de spray d'inhalation.

13. Procédé de fabrication d'un peptide selon l'une quelconque des revendications 1 à 7, comprenant les étapes suivantes :
a) faire réagir un précurseur de la fraction de liaison au virus avec un premier précurseur de la fraction de liaison à la mucine, le premier précurseur de la fraction de liaison à la mucine comprenant une première partie de la fraction de liaison à la mucine (1), afin d'obtenir un précurseur de peptide ; et
b) faire réagir le précurseur peptidique avec un deuxième précurseur de la fraction de liaison à la mucine, le deuxième précurseur de la fraction de liaison à la mucine comprenant une deuxième partie de la fraction de liaison à la mucine (1).

14. Procédé selon la revendication 13, **caractérisé en ce que** le précurseur de la fraction de liaison au virus comprend une fraction 4-pentynoïque liée de manière covalente à une fraction de liaison au virus (2).

15. Procédé selon la revendication 13 ou 14, **caractérisé en ce que** le premier précurseur de la fraction de liaison à la mucine comprend moins de la moitié de tous les acides aminés de la fraction de liaison à la mucine (1).
